# EUROPEAN PATENT SPECIFICATION

(11) **EP 4 048 232 B1**
(45) Date of publication and mention of the grant of the patent: **31.12.2025**
(21) Application number: 20792686.6
(22) Date of filing: 23.10.2020
(51) Int. Cl.: A61K 9/14, A61K 38/00, A61K 39/395, A61K 39/44, C07K 1/113

(54) **RNA OLIGONUCLEOTIDES FOR PREVENTING AGGREGATION OF PROTEINS**
RNA OLIGONUKLEOTIDE ZUR VORBEUGUNG VON PROTEINAGGREGATION
OLIGONUCLÉOTIDES D'ARN POUR EMPÊCHER L'AGRÉGATION DES PROTÉINES

(30) Priority: 23.10.2019 EP 19204799
(43) Date of publication of application: 31.08.2022
(73) Proprietor: Universität Wien, 1010 Vienna (AT)
(72) Inventor: ZAGROVIC, Bojan, 1030 Vienna (AT); POLYANSKY, Anton, 1030 Vienna (AT)
(74) Representative: Redl, Gerda
(86) International application number: PCT/EP2020/079882
(87) International publication number: WO 2021/078939

(56) References cited:
- WO-A2-2004/014306
- WO-A2-2016/009225
- MALIK RAVINDER ET AL: "Stabilization of bovine insulin against agitation-induced aggregation using RNA aptamers", INTERNATIONAL JOURNAL OF PHARMACEUTICS, ELSEVIER, NL, vol. 452, no. 1, 13 May 2013 (2013-05-13), pages 257 - 265, XP028573228, ISSN: 0378-5173, DOI: 10.1016/J.IJPHARM.2013.05.004
- ALAM PARVEZ ET AL: "Ascorbic acid inhibits human insulin aggregation and protects against amyloid induced cytotoxicity", ARCHIVES OF BIOCHEMISTRY AND BIOPHYSICS, ACADEMIC PRESS, US, vol. 621, 12 April 2017 (2017-04-12), pages 54 - 62, XP029991569, ISSN: 0003-9861, DOI: 10.1016/J.ABB.2017.04.005
- XIAOLING WANG ET AL: "Potential aggregation prone regions in biotherapeutics : A survey of commercial monoclonal antibodies", MABS, vol. 1, no. 3, 1 May 2009 (2009-05-01), US, pages 254 - 267, XP055410727, ISSN: 1942-0862, DOI: 10.4161/mabs.1.3.8035
- A. A. POLYANSKY ET AL: "Evidence of direct complementary interactions between messenger RNAs and their cognate proteins", NUCLEIC ACIDS RESEARCH ADVANCE ACCESS, vol. 41, no. 18, 18 July 2013 (2013-07-18), GB, pages 8434 - 8443, XP055675536, ISSN: 0305-1048, DOI: 10.1093/nar/gkt618

## Description

### FIELD OF THE INVENTION

The invention relates to the field of formulating polypeptides or proteins to increase solubility and/or prevent aggregation of the same, and designing suitable solubilizing RNA for such purpose.

### BACKGROUND OF THE INVENTION

The question of understanding and manipulating protein solubility in the context of high-protein-concentration aqueous solutions is a matter of both fundamental and practical relevance. On the one hand, with the cytoplasm of typical cells exhibiting protein concentrations of several 100s mg/mL, it is a fundamental problem to understand what the physicochemical determinants of protein solubility under such extreme circumstances are (Hetz et al. Nature Reviews Neuroscience 15, 233-249 (2014); Hipp et al. 2014, Trends in Cell Biology 24, 506-514; Patel et al. 2017, International Journal of Biological Macromolecules 100, 75-88). On the other hand, insufficient protein solubility is known to lead to protein unfolding, aggregation and loss of function and is associated with a number of pathogenic conditions such as amyloidogenic diseases and cancer. On a more practical side, high-protein-concentration preparations play an essential role in different industry applications such as, for example, the development of therapeutic formulations of monoclonal antibodies (mAbs) (Khalil et al. 2016, Nature Reviews Clinical Oncology 13, 273-290).

Safety and efficiency of biopharmaceutical proteins such as mAbs are hampered by their propensity to aggregate at high concentrations typically used in commercial formulations.

Patel et al. (2017) describe aptamers as stabilizers of monomeric proteins. Such aptamers are single stranded oligonucleotides (ssDNA/RNA) selected form a combinatorial library which bind to the target with high affinity, with an equilibrium dissociation constant (Kd) of aptamer-target complexes in pico- (or femto-) to micromolar range. By binding strongly to aggregation-prone surface loops of target proteins, such aptamers may also prevent their aggregation.

Chaudhary et al. (Molecular Therapy 2015, 23(12):1912-1926) describe selection of high-affinity RNA aptamers having specific three-dimensional structures from an RNA library against a monomeric mutant huntingtin to inhibit its aggregation.

Malik Ravinder et al. (International Journal f Pharmaceutics 2013, 452:257-265) describe stabilization of bovine insulin against agitation-induced aggregation using RNA aptamers comprising a folded tertiary structure, which aptamers bound to insulin with very low dissociation constants and high specificity due to their specific three-dimensional structure.

Hlevnjak et al, (Nucleic Acids Research 2012, 40(18):8874-8882) and Polyansky and Zagrovic (Nucleic Acids Research 2013, 41(18):8434-8443) describe direct complementary interactions between messenger RNAs and their cognate proteins and reveal statistically significant matching between composition of mRNA sequences and base-binding preferences of protein sequences they code for.

Zagrovic et al. (FEBS Letters 2018, 592:2901-2916) review experimental and computational work on RNA-protein interactions and describe how such interactions may be shaped by the intrinsic interaction affinities between individual nucleobases and protein side chains, claiming that the genetic code reflects the binding specificity between nucleobases and protein side chains.

WO2016/009225 A2 discloses G-quadruplex forming oligos solubilizing proteins, and re-folding aggregated proteins in the presence of intact rRNA comprising such G-quadruplex structure.

Parvez Alam et al. (Archives of Biochemistry and Biophysics 2017, 621:54-62) disclose ascorbic acid to inhibit human insulin aggregation.

Wang X. et al. (MABS 2009, 1(3):254-267) disclose potential aggregation prone regions in biotherapeutics.

WO2004/014306 A2 discloses treatment of diseases using oligonucleotides capable of disrupting protein aggregations *in situ* that are characteristic of disorders of protein assembly.

Development and administration of biopharmaceutical proteins faces a major challenge in that it typically requires formulations with high protein concentration (>100 mg/ml). The reasons for this include storage constraints, medically determined high-dosage requirements, practical demands of subcutaneous and/or sustained delivery and other. Importantly, a central challenge in developing high protein concentration formulations of therapeutics is the issue of protein solubility. Specifically, through a combination of electrostatic and hydrophobic intermolecular interactions, proteins at high concentration form aggregates, leading to a loss of pharmacological activity. Moreover, due to increased immunogenicity and toxicity, protein aggregates cause serious safety issues. The current approaches for addressing this challenge by different osmolytes and excipients suffer from various problems including insufficient protein activity, allergenic reactivity and others, and there exists an unmet need for novel strategies to increase the solubility of therapeutic protein products in pharmaceutical formulations in an efficient, cost-effective, target-specific manner.

There is a need for scientifically justified excipients in formulations of polypeptides or proteins, in particular biotechnological products.

### SUMMARY OF THE INVENTION

The objective is to improve methods of solubilizing therapeutic proteins and to prepare therapeutic protein formulations preventing unwanted aggregation even at high concentrations.

The objective is reached by the subject matter as claimed and as further described herein.

Herein, a novel class of protein-specific oligonucleotide excipients is provided.

Specifically, the invention provides for a composition comprising a protein and at least one single-stranded linear RNA oligonucleotide with an unfolded tertiary structure, which is characterized by:
a) a relative content of each of the nucleotides A, C, U, and G, which is the number of each of the nucleotides per total nucleotide number in the oligonucleotide, which relative content is the same ±10% (number of nucleotides per total nucleotide number) as compared to the relative content of the respective nucleotide in a reference; and/or
b) a relative content of at least 50% (number of nucleotides per total nucleotide number) of one type of nucleotide selected from the group consisting of nucleotides A, C, U, and G, which exhibits the highest relative content (number of each of the nucleotides per total nucleotide number) in a reference;

wherein the reference consists of a native mRNA or a set of different RNA sequences that covers all RNA sequences allowed by the universal genetic code, each encoding an amino acid sequence of at least three amino acids within a pre-determined aggregation-prone target region of said protein,
wherein the oligonucleotide has a length which is the same length as the reference, or longer,
wherein the solubility of said protein in an aqueous preparation is at least 20% increased compared to a formulation of said protein that does not comprise said oligonucleotide.

It is specifically understood that in a reference which is composed of a set of different RNAs, each of the RNAs encodes only one, in particular the same pre-determined aggregation-prone target region of said protein.

According to a specific embodiment, the relative content of any one, two, three, or four of the nucleotides A, C, U, or G is about the same (number of nucleotides per total nucleotide number) as compared to the relative content of the respective nucleotide in the reference.

The term "about the same" is herein understood as being the same ± any one of 10%, 9%, 8%, 7%, 6%, or 5%. Specifically, the percentage is understood as absolute percentage points from the relative fractions of different nucleotides.

According to an alternative specific embodiment, the selected type of nucleotide (A, C, U, or G) is most frequent in the reference.

Specifically, the relative content of the selected type of nucleotide (A, C, U, or G) in the oligonucleotide is at least any one of 50%, 60%, 70%, 80%, 85%, 90%, 95%, up to 100% (number of nucleotides per total nucleotide number).

Specifically, the oligonucleotide described herein is referred to as "solubilizing oligonucleotide".

According to a specific embodiment, the oligonucleotide is composed of only one type of nucleotide (A, C, U, or G), which is the selected one. Such an oligonucleotide, in which the relative content of a selected type of nucleotide (A, C, U, or G) is 100%, is herein also referred to as "homo-oligonucleotide" or "homo-oligo".

According to a specific aspect, the reference consists of only one molecule which is a native (herein also referred to as naturally-occurring) mRNA. Specifically, such reference consists of an mRNA, which is an mRNA molecule, or the nucleotide sequence information thereof, i.e. a virtual reference consisting of the mRNA nucleotide sequence information and the content of nucleotides A, C, U, and G contained therein, respectively.

Specifically, in the reference which is only one RNA, such as the native mRNA, the sum of the relative contents of the nucleotides A, C, U, and G amounts to 100%.

According to an alternative aspect, the reference consists of a set of different coding RNA sequences, each encoding the same aggregation-prone target region of said protein.

Specifically, the set is composed of a number of different coding RNA sequences, which is at least any one of 2, 3, 4, 5, 6, 7, 8, 9, 10, or higher, such as at least any one of 100, 500, 1000, 2000, 3000, 4000, or 5000, up to the number of coding RNAs theoretically allowed by the universal genetic code, herein also referred to as "all RNA sequences" encoding said pre-determined aggregation-prone target region of said protein.

Specifically, in the reference which is a set of different RNAs, the relative content of a nucleotide (selected from A, C, U, and G) is determined as the average relative content of the respective nucleotide in each of the RNAs comprised in the set. The average relative content of a type of nucleotide is specifically understood as the average of the value of the relative content of the respective nucleotide in each of the RNAs comprised in the set. Specifically, the sum of the average relative contents of the nucleotides A, C, U, and G amounts to 100%.

In particular, in a reference being a set of RNA sequences, each RNA comprised in the set differs in at least one nucleotide, and each of such RNAs encodes said pre-determined aggregation-prone target region of said protein. Specifically, such reference set of sequences consists of a collection of different RNA molecules, or the nucleotide sequence information thereof, i.e. a virtual reference consisting of a collection of RNA nucleotide sequence information, each characterizing a specific RNA, and the content of nucleotides A, C, U, and G contained therein, respectively. The average nucleotide content of such a set of sequences can be determined computationally from the average nucleotide content in codons of individual amino acids as given by the universal genetic code.

Specifically, in a reference set of sequences composed of a collection of RNA nucleotide sequences, the average relative content of each of the nucleotides A, C, U, and G, which is the number of each of the nucleotides per total nucleotide number, in the reference may be determined. Such average relative content of each of the nucleotides A, C, U, and G may be used as a reference to design and produce the suitable oligonucleotide which is characterized by a certain relative content of each of the nucleotides A, C, U, and G, or of a selected nucleotide.

In particular, the suitable oligonucleotide is characterized by
i. a relative content of each of the nucleotides A, C, U, and G, which is the number of each of the nucleotides per total nucleotide number in the oligonucleotide, which relative content is the same as said average relative content of the respective nucleotide ±10% (number of nucleotides per total nucleotide number); and/or
ii. a relative content of at least 50% (number of nucleotides per total nucleotide number), specifically, of one type of nucleotide selected from the group consisting of nucleotides A, C, U, and G, which is selected by comparing said average relative content of each of nucleotides A, C, U, and G, and selecting the one type of nucleotide with the highest average relative content.

Upon producing the suitable oligonucleotide, its solubilization or stabilization activity can be determined by producing a formulation of a protein comprising the pre-determined aggregation-prone target region which is targeted by said oligonucleotide, and determining the solubility and/or aggregation of said protein in an aqueous solution e.g., in a suitable storage stability study employing standard methods.

Specifically, the protein composition of formulation comprises at least two, three, four, five, six, seven, eight, nine or ten different single-stranded linear RNA oligonucleotides as described herein, wherein at least one of them is such oligonucleotide as further described herein, preferably two, three, four, five, six, seven, eight, nine or ten of them are such oligonucleotides as further described herein.

Specifically, said at least two different single-stranded linear RNA oligonucleotides are designed and provided, employing different references, or with respect to RNA sequences encoding different pre-determined aggregation-prone target region, in particular those, which are not overlapping regions, or non-contiguous sequences within said protein.

Specifically, said at least two different single-stranded linear RNA oligonucleotides are used in combination to solubilize said protein. Preferably, a combination of oligonucleotides is used, which additionally or synergistically increases the solubility and/or stability of the protein in aqueous solution as compared to the effect of only one of the individual oligonucleotides.

Specifically, the protein composition described herein comprises the protein and said at least one single-stranded linear RNA oligonucleotide (or the sum of said different oligonucleotides described herein) at a molar ratio ranging between 1:10 and 5:1, in particular at least any one of 1:10, 1:9, 1:8, 1:7, 1:6, 1:5, 1:4, 1:3, 1:2, 1:1; 2:1, 3:1, or 4:1.

Specifically, the oligonucleotide described herein is a ligand specifically targeting a protein, wherein the oligonucleotide interacts with the protein at the predetermined region of said protein.

Specifically, the oligonucleotide is an RNA molecule (herein also referred to as solubilizing RNA, solRNA) that has a length of at least 9 nt, up to e.g. 100 nt, e.g., specifically at least any one of 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 or 39, specifically at least any one of 40, 50, 60, 70, 80, 90, e.g. up to 100 nt.

Specifically, said oligonucleotide is a single stranded, linear oligonucleotide and their action as excipient does not rely of any particular three-dimensional structural fold. Therefore, said oligonucleotide specifically does not comprise an intramolecular loop structure, or loses such intramolecular loop structure in the presence of the protein.

Specifically, the oligonucleotide comprises at least any one of 50%, 60%, 70%, 80%, 85%, 90%, or 95% sequence identity to an mRNA encoding said protein, in particular compared to a naturally-occurring mRNA, or a fragment of any of the foregoing over a length of at least 9, 10, 15, 20, 25, or 30 nt.

Specifically, the oligonucleotide is an artificial RNA molecule, which is not naturally-occurring.

Specifically, the oligonucleotide is a non-coding RNA molecule, which lacks start or/and stop codons, specifically wherein said the oligonucleotide cannot be translated by the cellular machinery into an amino acid sequence.

Specifically, the solubilizing oligonucleotide is non-functional as an mRNA, and does not comprise an ORF (open reading frame), thus, are not coding or functionally not coding for the pre-determined aggregation-prone target region, or the respective protein.

Specifically, the solubilizing oligonucleotide is a fragment of a naturally-occurring mRNA (or a compositionally identical molecule), preferably comprising one or more mutations to render it non-coding.

Specifically, such mutations are selected from the group consisting of point mutations including frameshift mutations (deletion insertion) and/or substitution of one or more nucleobases).

Specifically, the number of point mutations as compared to a coding mRNA is limited such as e.g. to change less than half or one third of the nucleobases e.g., only 1, 2, 3, 4, 5, 6, 7, 8, 9, or 10 nucleobases in an oligonucleotide of a length of 20 and 30 nucleobases, respectively.

Specifically, one way to design an oligonucleotide as described herein is as follows: a set of oligonucleotides is selected from all possible mRNA fragments corresponding to one or more predetermined target regions in said protein e.g., those regions which are self-association prone, using a unique computational algorithm (such as reviewed in FEBS Letters 2018, 592:2901-2916) for direct assessment of oligonucleotide specificity to said protein. Specific embodiments refer to custom design of a variety of oligonucleotides, thereby obtaining a library of oligonucleotides, which is focused on a specific protein, and one or more suitable oligonucleotides are selected from such library for the purpose of improving solubility and preventing aggregation of said protein, as further described herein.

Specifically, binding of said oligonucleotide to said pre-determined aggregation-prone target region, or said protein (herein also referred to as "target" or "target protein") is non-covalent.

Specifically, binding of the oligonucleotide to its target is directly, i.e. without using an exogenous linker.

Specifically, binding of the oligonucleotide to its target is through electrostatic and van der Waals intermolecular interactions.

Specifically, binding of the oligonucleotide to its target is within a pre-determined target region of said protein, which has been identified to be solvent exposed and/or aggregation prone according to a computational prediction algorithm.

Specifically, the length of the oligonucleotide is at least the length of the nucleotide sequence that encodes the pre-determined aggregation-prone target region, or at least a fragment of said target region with a length of at least 3, 4, or 5 amino acids.

Specifically, the length of the oligonucleotide is at least the length of the reference that encodes an amino acid sequence of three amino acids within a pre-determined aggregation-prone region.

Specifically, the pre-determined target region consists of a peptide sequence or respective protein fragment, e.g., a peptide of at least 5 amino-acid residues, or at least 6, 7, 8, 9, 10, 11, 12, 13, 14, 15 amino-acid residues, up to the full length of said protein.

Specifically, the protein has a binding site recognizing a target binding partner, such as an antibody, an antigen, receptor, or ligand. The binding site may be composed of one or more linear or structural regions (e.g., loops, such as the CDR of an antibody) of said protein e.g., to form a binding surface or pocket. Specific proteins with a binding site are selected from antibodies, enzymes, signaling proteins, receptors, receptor ligands, peptide hormones, transport proteins, structural proteins, neurotransmitters, growth regulating factors, serum proteins, carriers, drugs, immunomodulators, oncogenes, tumor suppressors, toxins, and tumor antigens. Exemplary proteins with a binding site are antibodies or antigen-binding fragments of an antibody, or receptors such as T cell receptors. Binding of such protein to its target binding partner may trigger a biological response in a subject. Specifically, the protein comprises a binding site and has biological activity by binding the target binding partner at the binding site.

According to a specific embodiment, the protein has a binding site, and the predetermined target region, can be within said binding site e.g. a CDR of an antibody, or outside the binding site. Because the oligonucleotides described herein typically have at least order of magnitude lower affinity then target antigens, no interference is expected upon employing such oligonucleotides specifically targeting the antibody binding site.

Specifically, the protein is an antibody or an antigen-binding fragment thereof, such as a Fab fragment, and the predetermined region is within the CDR region or within the framework region of an antibody domain, e.g., within the VL and/or VH antibody domain, or within at least one of the constant antibody domains.

A pre-determined region of the target protein can be identified by methods well-known in the art. Specifically, said predetermined region is determined as follows: potential aggregation regions selected according to a computational protocol, which includes analysis of physicochemical profiles (e.g. hydrophobicity, aggregation score) of a said protein sequence using state-of-the-art algorithm or computer programs, and verification in direct molecular dynamics simulations of the self-association of said protein. For the latter purpose, a spatial model of a said protein structure is obtained from a protein databank (PDB) database or modeled using homology modeling techniques.

Specifically, said predetermined region is prone to aggregation, which is also herein referred to as "aggregation hotspots" or "sticky", which is characterized by tendency to promote aggregation of said protein in these sites. Usually such sticky region comprises or consists of a peptide sequence of at least 5 amino acid residues in length, or at least 6, 7, 8, 9, 10, 11, 12, 13, 14, 15 amino-acid residues. Such aggregation hotspots can be identified by methods known in the art e.g., employing certain rules, e.g. having high aggregation score, being exposed and/or being compositionally biased.

Specifically, the oligonucleotide is binding its target in a specific way, yet allowing cross-specificity along homologues protein targets with the sequence identity above 50%.

Specifically, said protein is a therapeutic (poly-)peptide or protein selected from a therapeutic (poly-)peptide or protein relacing an absent, deficient or mutated protein; a therapeutic (poly-)peptide or protein beneficial for treating inherited or acquired diseases, infectious diseases, or neoplasms (e.g. cancer or tumor diseases); an adjuvant or immuno-stimulating therapeutic (poly-)peptide or protein.

Specifically, said protein is a polypeptide or protein comprising or consisting of a human, chimeric, humanized and/or artificial amino acid sequence.

Specifically, the protein is a human protein.

Specifically, said protein has a structure which is the native tertiary and quarternary structure of the protein.

Specifically, the protein comprises a biological function when formulated with the oligonucleotide described herein.

Specifically, said protein comprises a biological function by interacting with reaction partners and/or ligands or receptors upon administering to a subject. Specifically, the biological function is obtained by a biological activity in vivo, and is typically determined in a suitable *ex vivo* assay employing the respective reaction partners and/or ligands or receptors. Specifically, the biological activity is any one or more of inhibiting, antagonizing or blocking the activity of endogenous compounds; or otherwise activating, agonizing or triggering the activity of endogenous compounds.

According to a specific embodiment, the protein is an antibody, and its biological function is obtained by interacting with its target antigen in the subject, thereby inducing an immune response.

According to a specific aspect, the specific biological function (unit per gram) is substantially the same, e.g., being at least any one of 50%, 55%, 60%, 65%, 70%, 75%, 80%, 85%, 90%, 95%, 100%, or at least 125%, or at least 150%, or at least 175%, or e.g. up to 200% of the activity compared to the same comparable protein in the same formulation, which is not provided with the solubilizing oligonucleotide(s) as described herein. The biological activity can even be higher than the comparable one, because of the solubilizing and/or stabilizing nature of the oligonucleotide in the protein composition or formulation.

Specifically, the protein composition described herein is provided in the isolated form or in an aqueous solution, in particular a pharmaceutical preparation.

Specifically, the protein composition is provided as an aqueous solution, frozen, or in the lyophilized form.

The invention further provides for an aqueous preparation comprising the protein composition of the invention, e.g., as a liquid, or in the frozen form, or an aqueous solution obtained upon reconstituting a lyophilized protein composition. Specifically, an aqueous solution is used for reconstitution purposes, which comprises said at least one single-stranded linear RNA oligonucleotide further described herein.

Therefore, the invention further provides for a kit comprising a protein lyophilizate and the aqueous reconstitution solution in separate containments, which reconstitution solution comprises said at least one single-stranded linear RNA oligonucleotide further described herein, to solubilize the protein upon reconstitution.

The invention further provides for a pharmaceutical preparation comprising the protein composition described herein and a pharmaceutically acceptable carrier.

Specifically, the pharmaceutical preparation comprises an aqueous formulation of the protein composition described herein.

Specifically, a pharmaceutical preparation is provided which is ready for use for administration to a subject in need thereof.

Specifically, the pharmaceutical preparation may be provided as a concentrate, e.g., in the frozen or lyophilized form, which may be reconstituted to an aqueous solution of the protein comprising the solubilizing oligonucleotide described herein.

Specifically, the subject is a human being, in particular a human being who is healthy or suffering from a disease.

Specifically, the pharmaceutical preparation or the aqueous preparation described herein comprises said protein at a concentration of at least any one of 0.1, 1, 10, 20, 30, 40, 50, 60, 70, 80, 90, or 100 mg/ml, specifically up to a saturating concentration.

Specifically, the protein contained in such pharmaceutical or aqueous preparation is characterized by an increased solubility in the presence of the solubilizing oligonucleotide described herein.

Specifically, in the pharmaceutical or aqueous preparation described herein, solubility of said protein is at least any one of 20%, 30%, 40%, 50%, 60%, 70%, 80%, 90%, or 100% (i.e. two-fold) increased as compared to a formulation of said protein that does not comprise said oligonucleotide. The increase of solubility may be determined by a solubility assay e.g., determining absorbance of soluble protein fraction, such as described in Example 2.

Specifically, in the pharmaceutical or aqueous preparation described herein, at least 85%, or at least 90%, or at least 95% of the protein is monodisperse, i.e. not aggregated. Monodispersity or the absence of aggregates may be determined by an aggregation assay e.g., by light-scattering measurements, such as described in Example 1 or 3.

Specifically, the pharmaceutical or aqueous preparation described herein does not comprise detectable aggregates of said protein in a static light scattering test as determined at a temperature of 37°C over at least 36 hours. In such experiments, the protein is typically tested at a concentration as used in pharmaceutical preparations e.g. within the range of 0.1 mg/mL to 200 mg/mL, such as e.g. at least 0.5, 1 mg/mL, 10 mg/mL or 100 mg/mL, preferably with the pH in the physiological range (pH = 7.4 +/- 0.2 or 0.1).

Specifically, the protein is characterized by an increased aggregation resistance in said preparation, if in contact with said oligonucleotide, such as in a composition comprising the protein and the oligonucleotide.

Specifically, in the pharmaceutical or aqueous preparation described herein, the aggregation resistance of said protein is determined by the amount of protein molecules in the native conformation and/or structure or fold of said protein present in the formulation, such that the amount of unspecific aggregates or inactive proteins in an aqueous formulation is less than any one of 10%, 9%, 8%, 7%, 6%, 5%, 4%, 3%, or 2%, compared to a formulation of said protein that does not comprise said oligonucleotide.

Specifically, in the pharmaceutical or aqueous preparation described herein, the oligonucleotide concentration is at least any one of 10, 20, 30, 40, or 50 µM, and the protein concentration is at least any one of 10, 20, 30, 40, or 50 µM.

Specifically, the pharmaceutical preparation described herein comprises a pharmaceutically acceptable carrier, preferably suitable for a parenteral or mucosal formulation. Specifically, the parenteral formulation is provided for i.v., s.c., or i.m. use.

Typically, the pharmaceutical preparation described herein does not require a further excipient (such as citrate, polysorbate, sugars, or amino acids) because of the solubilizing oligonucleotide contained therein. Therefore, the pharmaceutical preparation may be provided without any excipient other than oligonucleotide(s).

According to a specific embodiment, the pharmaceutical preparation described herein may be provided as a mixture of the oligonucleotide(s) and the protein, in particular a mixture that allows interacting prior to use, or as kit of parts, wherein at least one of the oligonucleotides and the target protein is provided in separate formulations or containments (separate component preparations) that can be combined prior to use to provide the pharmaceutical preparation. In particular, the component preparation comprising said at least one oligonucleotide and the component preparation comprising said target protein can be provided in one or two separate containments.

A kit of parts described herein may include, in addition to the respective components, various other therapeutic agents and auxiliary agents and devices to prepare a pharmaceutical formulations ready-for-use. A kit may also include instructions for use in a therapeutic method. Such instructions can be, for example, provided on a device included in the kit. In another specific embodiment, the kit includes one component comprising the protein in the lyophilized form, and another component comprising the oligonucleotide(s) in an aqueous solution that can be mixed before use to reconstitute the lyophilizate and to produce a pharmaceutical preparation for near term administration.

The invention further provides for a method of preparing the protein composition described herein, comprising mixing said protein with said oligonucleotide(s).

Specifically, at least one of said oligonucleotides is obtained by a method comprising:
a) identifying an aggregation-prone target region of said protein;
b) determining the average relative content of each of the nucleotides A, C, U, and G, which is the number of each of the nucleotides per total nucleotide number, in a reference which is a set of different RNA sequences, each encoding said target region; and
c) designing and producing an oligonucleotide which is characterized by
   i. a relative content of each of the nucleotides A, C, U, and G, which is the number of each of the nucleotides per total nucleotide number in the oligonucleotide, which relative content is the same as said average relative content of the respective nucleotide ±10% (number of nucleotides per total nucleotide number); and/or
   ii. a relative content of at least 50% (number of nucleotides per total nucleotide number) of one type of nucleotide selected from the group consisting of nucleotides A, C, U, and G, which is selected by comparing said average relative content of each of nucleotides A, C, U, and G, and selecting the one type of nucleotide with the highest average relative content.

According to a specific aspect, said at least one of said oligonucleotides is produced by synthesizing a fragment of an mRNA encoding said target region and optionally modifying the sequence of said mRNA fragment. Such synthesis is herein also understood as a process of rational design considering direct complementarity between mRNA and the cognate (or encoded) protein, and the respective interactions that allow coating or binding of RNA oligonucleotides to the predetermined region.

Specifically, the oligonucleotide is designed using an algorithm considering specific complementary interactions between nucleotides and amino acids in the predetermined target region.

Specifically, the method comprises determining sequences of an oligonucleotide and the target protein, and in particular the predetermined target region of the protein.

According to a specific method, one or more predetermined regions are identified and the respective reference for oligonucleotide design is constructed; and one or more oligonucleotides are virtually screened against sequence of a said protein, wherein the virtual screening process identifies putative oligonucleotide hits.

Specifically, the method further comprises testing one or more of the oligonucleotide hits from the virtual screen using an experimental assay for solubility tests.

According to a specific aspect, the invention provides for the use of at least one single-stranded linear RNA oligonucleotide comprised in the composition of the invention as an excipient in an aqueous protein solution, wherein the oligonucleotide is used in an amount sufficient to prevent aggregation of the protein to less than 10% aggregates when stored in solution.

Specifically, the oligonucleotide may be used as an excipient in a protein preparation that is provided in a storage-stable form, such as frozen or lyophilized. Upon thawing and reconstituting, respectively, a composition can be provided as further described herein.

Specifically, the oligonucleotide does not comprise a folded structure, such as further described herein.

Specifically, the oligonucleotide is structurally dynamic, i.e. without a pre-determined structure that would allow high affinity binding to said protein, such as an aptamer.

Specifically, such oligonucleotide is used as an excipient in a protein preparation, which is characterized as further described herein, in particular wherein the oligonucleotide is characterized by:
a) a relative content of each of the nucleotides A, C, U, and G, which is the number of each of the nucleotides per total nucleotide number in the oligonucleotide, which relative content is the same ±10% (number of nucleotides per total nucleotide number) as compared to the relative content of the respective nucleotide in a reference; and/or
b) a relative content of at least 50% (number of nucleotides per total nucleotide number) of one type of nucleotide selected from the group consisting of nucleotides A, C, U, and G, which is selected by comparing the relative content of each of nucleotides A, C, U, and G, which is the number of each of the nucleotides per total nucleotide number of a reference, and selecting the one type of nucleotide with the highest relative content;
wherein the reference consists of a native mRNA or a set of different RNA sequences, each encoding a pre-determined aggregation-prone target region of said protein.

Specifically, such oligonucleotide is used as an excipient in a protein preparation, wherein the reference consists of a native mRNA or a set of different RNA sequences that covers all RNA sequences allowed by the universal genetic code, each encoding an amino acid sequence of at least three amino acids within a pre-determined aggregation-prone target region of said protein, and/or
wherein the oligonucleotide has a length which is the same length as the reference, or longer.

Specifically, the oligonucleotide is used in an amount sufficient to prevent aggregation of the protein to less than 10%, 9%, 8%, 7%, 6%, 5%, 4%, 3%, or 2%, aggregates when stored in solution.

Therefore, a unique and innovative solution to the problem underlying the invention is given by providing solubilizing RNAs (also referred to as "solRNAs") to target aggregation hotspots of a protein and thus preventing unwanted aggregation. Specific examples have been prepared using *in-silico* rational design of such solubilizing RNAs.

According to specific examples, exemplary proteins and aggregation prone peptide fragments thereof, such as human insulin and a monoclonal IgG1 antibody (e.g., trastuzumab (Herceptin, Roche)), have been solubilized using one or more different solubilizing oligonucleotides designed and produced according to the method described herein, and the change in solubility or aggregation behavior has been determined.

### FIGURES

Figure 1: Design and effect of two mRNA fragments on the native insulin solubility. In both cases, protein and RNA concentrations were 86 µM and 20 µM, respectively. RNA1: SEQ ID NO:4; RNA2: SEQ ID NO:5.

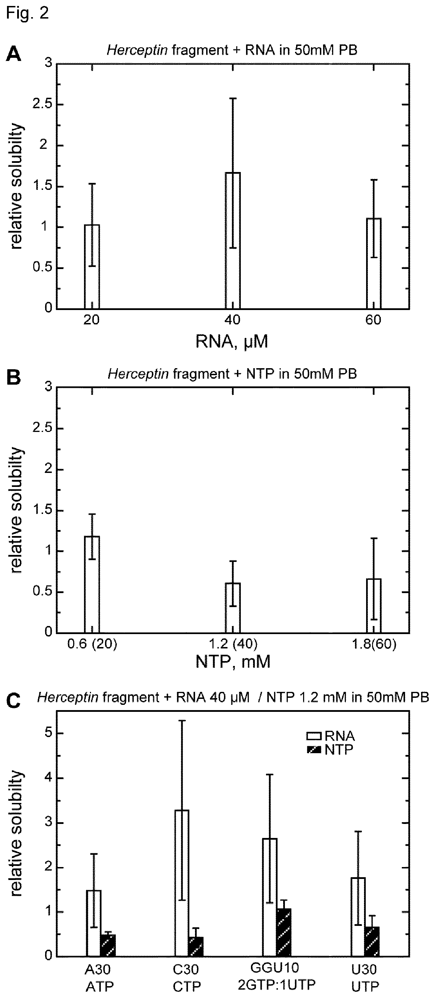

Figure 2: RNA oligonucleotides significantly outperform the respective solutions of single nucleotides (NTP) with equivalent total nucleotide concentration in solubilizing Herceptin fragment LLIYSASFLY (SEQ ID NO:1). **A-B:** Average solubility change upon titration of the Herceptin fragment using **(A)** RNAs or **(B)** single nucleotides, with the corresponding RNA concentrations given in units of µM in the parentheses. On average, the optimum concentration for RNAs was found to be at 40 µM. **C:** The effect of 30 nt homo-oligos at 40 µM as compared to the equivalent composition of single nucleotides at the same effective concentration of nucleotides. See Tables 3 and 4 for details.
Figure 3: The solubility increase for the *Herceptin* fragment LLIYSASFLY (SEQ ID NO:1) depends on oligonucleotide length. Data is shown for homo C oligonucleotide.
Figure 4: Solubility increase for the *Herceptin* fragment LLIYSASFLY (SEQ ID NO:1) by its own mRNAs depends on their compositional bias towards pyrimidine-rich sequences. The tested RNAs were derived from a pool of all possible mRNAs coding for the *Herceptin* fragment by ranking their affinity to the peptide as calculated using a computational algorithm (referred to as SOLYRNA).
Figure 5: Sequence alignment for the tested mRNAs of the *Herceptin* fragment LLIYSASFLY (SEQ ID NO:1); upper sequence (161811U): SEQ ID NO:2; lower sequence (Native mRNA): SEQ ID NO:3.
Figure 6: The used computational algorithm is able to rank properly the solubilization effects of different RNAs. A linear regression between the calculated affinity score and the logarithm of the relative solubility as measured for the *Herceptin* fragment LLIYSASFLY (SEQ ID NO:1) in the presence of different RNAs at **(A)** 20, **(B)** 40, and (C) 60 µM.
Figure 7:
   **Table 3.** Effect of RNA oliogos on solubilty of a *Herceptin* fragment (LLIYSASFLY, SEQ ID NO:1)
Figure 8:
   **Table 4.** Effect of single nucleotides on the solubility of a *Herceptin* fragment (LLIYSASFLY, SEQ ID NO:1)
Figure 9.
   Aggregation studies for the tested RNA oligos **(A),** Herceptin and Herceptin compositions with the oligos **(B)** the using static light scattering (SLS) technique. Normalized intensities are proportional to the mass of aggregates in tested samples. A growth of aggregate masses (an increase in intensity) is not observed for Herceptin in a composition with the tested RNA oligos (each individually), while the protein alone displays aggregation during 36 h of the experiment.

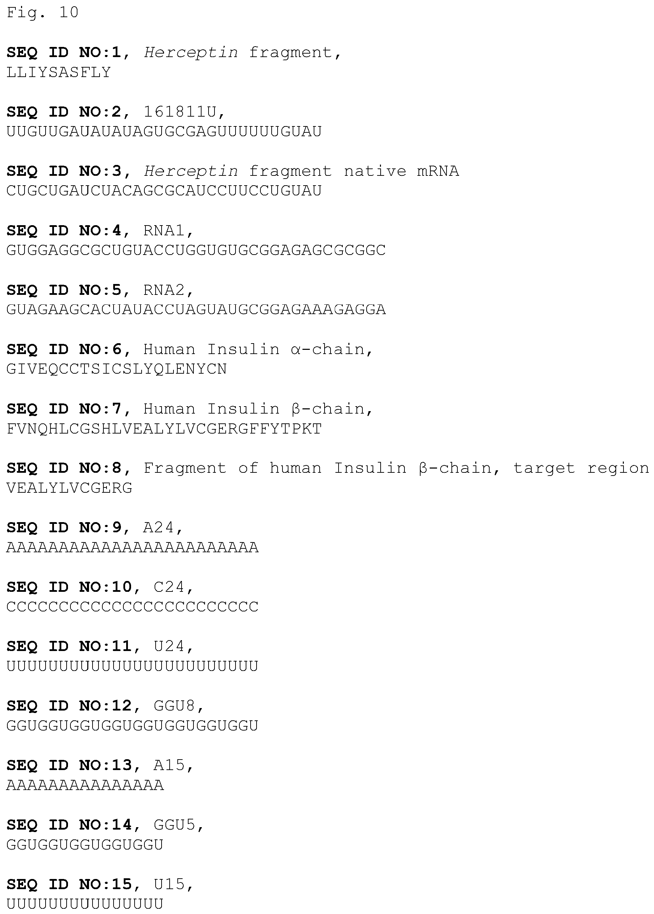

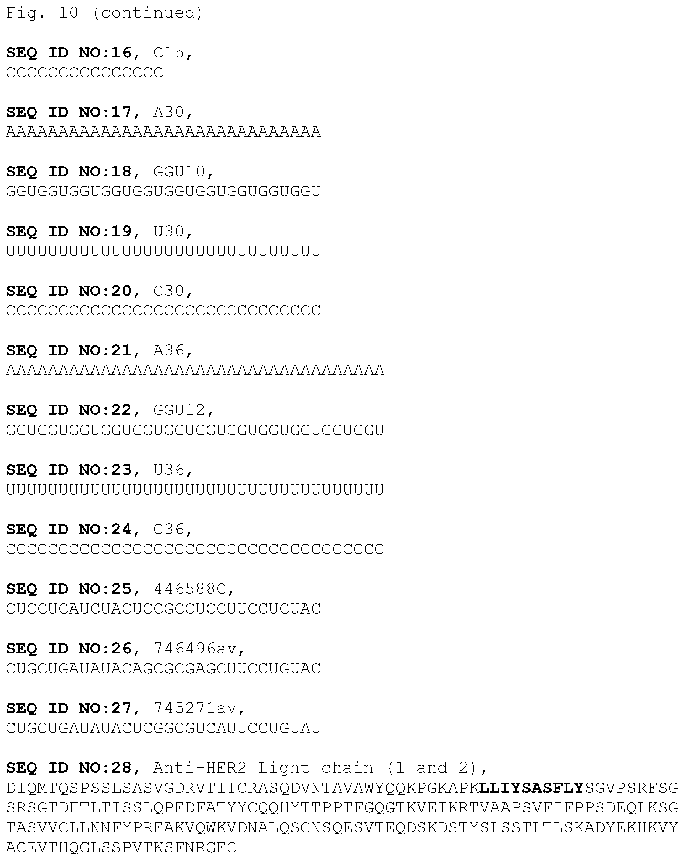

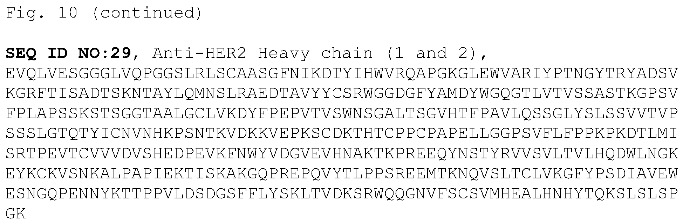

Figure 10: Sequences referred to herein:
   **SEQ ID NO:1,** Herceptin fragment
   **SEQ ID NO:2,** 161811U
   **SEQ ID NO:3,** *Herceptin* fragment native mRNA
   **SEQ ID NO:4,** RNA1
   **SEQ ID NO:5,** RNA2
   **SEQ ID NO:6,** Human Insulin α-chain
   **SEQ ID NO:7,** Human Insulin β-chain
   **SEQ ID NO:8,** Fragment of human Insulin β-chain, target region
   **SEQ ID NO:9,** A24
   **SEQ ID NO:10,** C24
   **SEQ ID NO:11,** U24
   **SEQ ID NO:12,** GGU8
   **SEQ ID NO:13,** A15
   **SEQ ID NO:14,** GGU5
   **SEQ ID NO:15,** U15
   **SEQ ID NO:16,** C15
   **SEQ ID NO:17,** A30
   **SEQ ID NO:18,** GGU10
   **SEQ ID NO:19,** U30
   **SEQ ID NO:20,** C30
   **SEQ ID NO:21,** A36
   **SEQ ID NO:22,** GGU12
   **SEQ ID NO:23,** U36
   **SEQ ID NO:24,** C36
   **SEQ ID NO:25,** 446588C
   **SEQ ID NO:26,** 746496av
   **SEQ ID NO:27,** 745271av
   **SEQ ID NO:28,** Anti-HER2 Light chain (1 and 2)
   **SEQ ID NO:29,** Anti-HER2 Heavy chain (1 and 2)

### DETAILED DESCRIPTION

Unless indicated or defined otherwise, all terms used herein have their usual meaning in the art, which will be clear to the skilled person. Reference is for example made to the standard handbooks, such as Sambrook et al, "Molecular Cloning: A Laboratory Manual" (2nd Ed.), Vols. 1 -3, Cold Spring Harbor Laboratory Press (1989); Lewin, "Genes IV", Oxford University Press, New York, (1990), and Janeway et al, "Immunobiology" (5th Ed., or more recent editions), Garland Science, New York, 2001.

The subject matter of the claims specifically refers to artificial products or methods employing or producing such artificial products, which may be variants of native (wild-type) products. Though there can be a certain degree of sequence identity to the native primary structure of the cognate mRNA of a given target peptide sequence, it is well understood that the materials, methods and uses of the invention, e.g., specifically referring to isolated nucleic acid sequences, amino acid sequences, complexes of polypeptides or proteins coated with one or more oligonucleotides, and proteins modified by complexing with one or more oligonucleotides, are "man-made" or synthetic, and are therefore not considered as a result of "laws of nature".

The oligonucleotide which is provided as solubilizing RNA and e.g. used as excipient in the protein composition described herein is herein understood as an artificial nucleic acid molecule which does not occur naturally in the context of binding the target protein. Such nucleic acid molecule may be non-natural due to its individual sequence (which does not occur naturally) and/or due to other modifications, e.g. chemical modifications of standard nucleotides, which do not occur naturally, i.e. they differ from any wild type sequence by at least one nucleotide. The term "wild type" may be understood as a sequence occurring in nature.

The term "coding" as used herein in particular with respect to an mRNA is understood as follows. The skilled person is aware that in order to determine whether an RNA sequence is coding, the RNA sequence can be translated to the amino acid sequence of a peptide or protein; or can be indirectly converted to a protein sequence e.g., in a first step converted into the corresponding DNA sequence (in particular by replacing the uracils (U) by thymidines (T) throughout the sequence). Thereafter, the genetic code of the DNA sequence can be translated to the respective amino acid sequences. A series of different RNA codons can translate into the same amino acid, can correspond to a variety of DNA codons which may translate into the same amino acid. This is because of synonymous codons that, despite a small mutation, can code for the same amino acid.

The term "derived from" as used herein in particular in the context of an artificial oligonucleotide derived from a coding mRNA, shall mean that the (artificial) oligonucleotide, shares a certain compositional identity with the mRNA from which it is derived. Specific embodiments described herein refer to the design of oligonucleotides that are derived from mRNA (or mRNA fragments) encoding a target protein.

Though the solubilizing oligonucleotide described herein may consist of or be derived from a fragment of a full-length mRNA encoding the protein, it can of course also of the full-length mRNA or be derived from the full-length mRNA.

The term "fragment" in the context of a nucleic acid molecule or nucleic acid sequences and in particular mRNA sequences refers to a continuous subsequence of the full-length reference (or "parent") nucleic acid sequence. In other words, a "fragment" is typically a shorter portion of a full-length nucleic acid sequence. Accordingly, a fragment, typically, consists of a sequence that is identical to the corresponding stretch within the full-length nucleic acid sequence. The term includes naturally occurring fragments as well as engineered fragments.

Specifically, the pharmaceutical or aqueous preparation described herein comprises the protein composition described herein in the isolated form.

The term "isolated" or "isolation" as used herein with respect to a nucleic acid, a target protein or a protein composition comprising target protein and a solubilizing oligonucleotide described herein shall refer to such composition that has been sufficiently separated from the environment with which it would naturally be associated, so as to exist in "substantially pure" form. "Isolated" does not necessarily mean the exclusion of artificial or synthetic mixtures with other compounds or materials, or the presence of impurities that do not interfere with the fundamental activity, and that may be present, for example, due to incomplete purification. Isolated compounds can be formulated with diluents or adjuvants and still for practical purposes be isolated - for example, the polypeptides or polynucleotides can be mixed with pharmaceutically acceptable carriers when used in diagnosis or therapy.

The term "protein" or "target protein" as used herein shall refer to any of (poly)peptides or proteins of interest in their naturally occurring (wild-type) form, as well as variants, fragments and derivatives thereof. Specific target proteins are human, or artificial proteins, including without limitation chimeric or humanized versions of human proteins. Without being limited thereto, target proteins can be therapeutic, antigenic and allergenic (poly-)peptides. Specific target proteins are plasma proteins or plasma fractions comprising proteins, such as immunoglobulins, blood factors, or blood coagulation factors. Specific further target proteins are small proteins like peptides or polypeptides, e.g. of at least 20 or 30 amino acid length, such as insulin, or glucagon-like peptide 2.

The term "therapeutic protein" as used herein shall refer to a (poly-)peptide or protein capable of mediating a desired diagnostic, prophylactic or therapeutic effect, preferably resulting in detection, prevention, amelioration and/or healing of a disease.

A specific target protein is an antigen-binding molecule such as an antibody, or a fragment thereof, in particular an antibody fragment comprising an antigen-binding domain. Among specific target proteins are antibodies such as monoclonal antibodies (mAbs), immunoglobulin (Ig) or immunoglobulin class G (IgG, in particular IgG1), heavy-chain antibodies (HcAb's), or fragments thereof such as fragment-antigen binding (Fab), Fd, single-chain variable fragment (scFv), or engineered variants thereof such as for example Fv dimers (diabodies), Fv trimers (triabodies), Fv tetramers, or minibodies and single-domain antibodies like VH, VHH, IgNARs, or V-NAR, or any protein comprising an immunoglobulin-fold domain. Further antigen-binding molecules may be selected from antibody mimetics, or (alternative) scaffold proteins such as e.g., engineered Kunitz domains, Adnectins, Affibodies, Affiline, Anticalins, or DARPins.

The term "antibody" as used herein is defined as antigen-binding polypeptides that are either immunoglobulins or immunoglobulin-like molecules e.g., composed of one or more antibody domains and bearing antigen-binding properties similar to immunoglobulins or antibodies, in particular proteins that may exhibit mono- or bi- or multi-specific, or mono-, bi- or multivalent binding properties, e.g. at least two specific binding sites for epitopes of antigens, effector molecules or structures, specifically of pathogen origin or of human structure, like self-antigens including cell-associated or serum proteins.

An antibody typically consists of or comprises antibody domains, which are understood as constant and/or variable domains of the heavy and/or light chains of immunoglobulins, with one or more or without a linker sequence. Antibodies are specifically understood to consist of or comprise combinations of variable and/or constant antibody domains with or without a linking sequence or hinge region, including pairs of variable antibody domains, such as one or two VH/VL pairs. Polypeptides are understood as antibody domains, if comprising a beta-barrel structure consisting of at least two beta-strands of an antibody domain structure connected by a loop sequence. Antibody domains may be of native structure or modified by mutagenesis or derivatization, e.g. to modify the antigen binding properties or any other property, such as stability or functional properties, such as binding to the Fc receptors FcRn and/or Fcgamma receptor.

The term "antibody" as used herein specifically includes full-length antibodies, including antibodies of immunoglobulin-like structures. Specifically, an antibody can be a full-length antibody, e.g. of an IgG type (e.g., an IgG1, IgG2, IgG3, or IgG4 subtype), IgA1, IgA2, IgD, IgE, or IgM antibody.

The term further includes any of derivatives, combinations or fusions of antibodies, antibody domains, or antibody fragments.

The term "full length antibody" is used to refer to any antibody molecule comprising an antigen-binding region and an Fc region or at least most of the Fc part of an antibody, which specifically includes a dimer of heavy chains. This term "full length antibody" is used herein to emphasize that a particular antibody molecule is not an antibody fragment.

In accordance therewith, an antibody is typically understood as a protein (or protein complex) that includes one or more polypeptides substantially encoded by immunoglobulin genes or fragments of immunoglobulin genes. The recognized immunoglobulin genes include the kappa, lambda, alpha, gamma, delta, epsilon, and mu constant region genes, as well as immunoglobulin variable region genes. Light chains (LC) are classified as either kappa (including a VL and a Clambda domain) or lambda (including a VL and a Ckappa domain). Heavy chains (HC) are classified as gamma, mu, alpha, delta, or epsilon, which in turn define the immunoglobulin classes, IgG, IgM, IgA, IgD and IgE, respectively.

A HC or LC is each composed of at least two domains connected to each other to produce a chain of domains. It is specifically understood that an antibody HC includes a VH antibody domain and at least one antibody domain C-terminally bound to the VH, i.e. the at least one antibody domain is connected to the C-terminus of the VH domain with or without a linking sequence. An antibody LC includes a VL antibody domain and at least one antibody domain C-terminally bound to the VL, i.e. the at least one antibody domain is connected to the C-terminus of the VL domain with or without a linking sequence.

The definition further includes domains of the heavy and light chains of the variable region (such as dAb, Fd, VI, Vk, Vh, VHH) and the constant region or individual domains of an intact antibody such as CH1, CH2, CH3, CH4, Cl and Ck, as well as mini-domains consisting of at least two beta-strands of an antibody domain connected by a structural loop. Typically, an antibody having an antigen-binding site through a specific CDR structure is able to bind a target antigen through the CDR loops of a pair of VH/VL domains.

The term "antibody" shall apply to antibodies of animal origin, including human species, such as mammalian, including human, murine, rabbit, goat, camelid, llama, cow and horse, or avian, such as hen, which term shall particularly include recombinant antibodies which are based on a sequence of animal origin, e.g. human sequences.

The term "antibody" further applies to chimeric antibodies, e.g. chimeric antibodies, with sequences of origin of different species, such as sequences of murine and human origin.

The term "antibody" may further apply to humanized antibodies.

The term "antibody" further applies to monoclonal or polyclonal antibodies, specifically a recombinant antibody, which term includes all antibodies and antibody structures that are prepared, expressed, created or isolated by recombinant means, such as antibodies originating from animals, e.g. mammalians including human, that comprises genes or sequences from different origin, e.g. chimeric, humanized antibodies, or hybridoma derived antibodies. Further examples refer to antibodies isolated from a host cell transformed to express the antibody, or antibodies isolated from a recombinant, combinatorial library of antibodies or antibody domains, or antibodies prepared, expressed, created or isolated by any other means that involve splicing of antibody gene sequences to other DNA sequences.

A specific therapeutic protein is a blood coagulation factor, in particular a human blood coagulation factor, such as any of Factor VIII, one or more factors of a prothrombin complex, or Factor XIII.

A specific therapeutic protein is a hormone, including any pre- or preforms of a hormone, in particular a human hormone, such as any of insulin or glucagon.

A specific target protein is a vaccine antigen, which is herein understood as an antigen of subunit vaccines, such as comprising or consisting of an antigenic (poly)peptide or protein, and are specifically understood as comprising, consisting of or being capable of providing at least one (functional) epitope. A "functional" epitope refers to an epitope capable of inducing a desired adaptive immune response in a subject.

Exemplary vaccine antigens are selected from the group consisting of tumor antigens (such as derived from or associated with a (malignant) tumor or a cancer disease), pathogenic antigens (such as derived from or associated with pathogens, i.e. viruses, microorganisms, or other substances causing infection), autoantigens (such as endogenous self-antigens (endogenous to a host, such as a human being) that induce an autoimmune reaction in the host), or allergens or allergenic antigens (such as antigens capable of inducing an allergic reaction, i.e. a pathological immunological reaction characterized by hypersensitivity or adverse immunological reactions involving immunoglobulin E (IgE) upon exposure to a subject).

The term "excipient" as used herein shall refer to facilitate formulation of a compound such as a protein into a composition, such as a pharmaceutical preparation. Excipients are typically pharmaceutically acceptable substances which are understood as being are non-toxic and otherwise biologically suitable for administration to a subject (like a human being). Such excipients facilitate administration of the compounds described herein and are compatible with the active ingredient. Examples of pharmaceutically-acceptable excipients include stabilizers, lubricants, surfactants, diluents, anti-oxidants, binders, coloring agents, bulking agents, emulsifiers, or taste-modifying agents.

The oligonucleotides described herein are specifically used as excipients in a protein preparation.

According to specific aspects, excipient compounds as disclosed herein can suppress protein aggregation or clustering due to specific interactions between the excipient compound and the target protein in solution. Excipient compound as disclosed herein can be added to a protein preparation in a solubility-increasing amount or in a stability-increasing amount, in particular an amount that prevents protein aggregation or polydispersity of the particle size distribution in the protein preparation.

The effect of an oligonucleotide excipient on the protein preparation as described herein can e.g. be measured by turbidity, light scattering, or particle counting analysis.

Specific aspects described herein refer to oligonucleotides used as excipients to render a protein preparation resistant to an increase in protein particle size or proteinacous particle size, as measured by dynamic light scattering (DLS) analysis. In DLS analysis, the size of suspended particles can be observed as a median particle diameter. Ideally, the median particle diameter of the protein should be relatively unchanged since the particle diameter represents the active component in the monomeric state, as opposed to aggregated (dimeric, trimeric, oligomeric, etc.) or fragmented states. An increase of the median particle diameter, therefore, can represent an aggregated protein. Thus, the stability of a protein preparation can be observed by the relative change in median particle diameter after an elapsed time. According to specific aspects, the protein preparations as disclosed herein are resistant to forming a polydisperse particle size distribution as measured by dynamic light scattering (DLS) analysis. Therefore, a protein preparation can contain a monodisperse particle size distribution of colloidal protein particles. In specific embodiments, a stability result is to have less than a 10% change in the median particle diameter compared to the initial median particle diameter of the protein preparation described herein. In specific embodiments, an improvement in stability of a protein preparation described herein can be measured as a lower percent change of the median particle diameter after a certain elapsed time, as compared to the median particle diameter in a control preparation that does not contain the excipient. In specific embodiments, an improvement in stability of a protein preparation described herein as disclosed herein can be measured as a lower percent change of the median particle diameter after exposure to a stress condition, as compared to the percent change of the median particle diameter in a control preparation that does not contain the excipient. In other words, improved stability can prevent an increase in particle size as measured by light scattering. The stress conditions can be a low-temperature storage, high-temperature storage, exposure to air, exposure to gas bubbles, exposure to shear conditions, or exposure to freeze/thaw cycles. In specific embodiments, an improvement in stability of a protein preparation as disclosed herein can be measured as a less poly disperse particle size distribution as measured by DLS, as compared to the polydispersity of the particle size distribution in a control preparation that does not contain the excipient.

The term "oligonucleotide" as used herein shall refer to RNA molecules of a certain length. Preferably, an oligonucleotide is a polymer comprising or consisting of nucleotide monomers (the variable parts of which are called nucleobases), which are covalently linked to each other by phosphodiester-bonds of a sugar/phosphate-backbone. The term "oligonucleotide" also encompasses modified nucleic acid molecules, such as base-modified, sugar-modified or backbone- modified etc. RNA molecules.

The term "sequence identity" as used herein is understood as the relatedness between two amino-acid sequences or between two nucleotide sequences and described by the degree of sequence identity. The sequence identity of a variant, homologue or orthologue as compared to a parent nucleotide or amino acid sequence indicates the degree of identity of two or more sequences. Two or more amino-acid sequences may have the same or conserved amino acid residues at a corresponding position, to a certain degree, up to 100%. Two or more nucleotide sequences may have the same or conserved base pairs at a corresponding position, to a certain degree, up to 100%.

Sequence similarity searching is an effective and reliable strategy for identifying homologs with excess (e.g., at least 50%) sequence identity. Sequence similarity search tools frequently used are e.g., BLAST, FASTA, and HMMER.

Sequence similarity searches can identify such homologous proteins or polynucleotides by detecting excess similarity, and statistically significant similarity that reflects common ancestry. Homologues may encompass orthologues, which are herein understood as the same protein in different organisms, e.g., variants of such protein in different different organisms or species.

"Percent (%) identity" with respect to an amino-acid sequence, homologs and orthologues described herein is defined as the percentage of amino-acid residues in a candidate sequence that are identical with the amino-acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

For purposes described herein, the sequence identity between two amino acid sequences is determined using the NCBI BLAST program version 2.2.29 (Jan-06-2014) with blastp set at the following exemplary parameters: Program: blastp, Word size: 6, Expect value: 10, Hitlist size: 100, Gapcosts: 11.1, Matrix: BLOSUM62, Filter string: F, Genetic Code: 1, Window Size: 40, Threshold: 21, Composition-based stats: 2.

"Percent (%) identity" with respect to a nucleotide sequence e.g., of a nucleic acid molecule or a part thereof, in particular a coding DNA or RNA sequence, is defined as the percentage of nucleotides in a candidate DNA or RNA sequence that is identical with the nucleotides in the DNA sequence, after aligning the sequence and introducing gaps, if necessary, to achieve the maximum percent sequence identity, and not considering any conservative substitutions as part of the sequence identity. Alignment for purposes of determining percent nucleotide sequence identity can be achieved in various ways that are within the skill in the art, for instance, using publicly available computer software. Those skilled in the art can determine appropriate parameters for measuring alignment, including any algorithms needed to achieve maximal alignment over the full length of the sequences being compared.

Optimal alignment may be determined with the use of any suitable algorithm tor aligning sequences, non-limiting examples of which include the Smith-Waterman algorithm, the Needleman-Wunsch algorithm, algorithms based on the Burrows-Wheeler Transform (e.g., the Burrows Wheeler Aligner), ClustalW, Clustal X, BLAT, Novoalign (Novocraft Technologies; available at novocraft.com), ELAND (Illumina, San Diego, CA), SOAP (available at soap.genomies.org.cn), and Maq (available at maq.sourceforge.net).

The term "protein" as used herein is herein understood as a (poly)peptide or protein of interest that can be encoded by an mRNA, and can either be isolated from its natural environment, or be expressed under suitable conditions to yield a functional (poly)peptide or protein product. In this context, the term "functional" means "capable of exerting a desired biological function" and/or "exhibiting a desired biological property". (Poly)peptides or proteins of interest can have various functions and include, for instance, antibodies, enzymes, signaling proteins, receptors, receptor ligands, peptide hormones, transport proteins, structural proteins, neurotransmitters, growth regulating factors, serum proteins, carriers, drugs, immunomodulators, oncogenes, tumor suppressors, toxins, tumor antigens, and others. These proteins can be post-translationally modified to be proteins, glycoproteins, lipoproteins, phosphoproteins, etc.

Specifically, the protein composition described herein comprises the protein in substantially pure form.

The term "substantially pure" or "purified" as used herein shall refer to a compound or preparation comprising at least 50% (w/w), preferably at least 60%, 70%, 80%, 90% or 95% of a compound. Purity is measured by methods appropriate for the compound (e.g. chromatographic methods, polyacrylamide gel electrophoresis, HPLC analysis, and the like).

The term "subject" as used herein shall refer to a warm-blooded mammal, particularly a human being or a non-human animal. Thus, the term "subject" may also particularly refer to animals including dogs, cats, rabbits, horses, cattle, pigs and poultry. In particular the protein composition described herein is provided for medical use to treat a subject or patient in need of prophylaxis or treatment of a disease condition. The term "patient" includes human and other mammalian subjects that receive either prophylactic or therapeutic treatment. The term "treatment" is thus meant to include both prophylactic and therapeutic treatment.

An oligonucleotide as used herein for the purpose of solubilizing a target protein of interest can be designed as a variant of an RNA sequence (used as a "parent" sequence) that encodes the target protein or a predetermined part thereof.

The term "variant" as used herein in the context of nucleic acid sequences refers to nucleic acid sequence variants, i.e. nucleic acid sequences that differ in at least one nucleic acid from a reference (or "parent") nucleic acid sequence of a reference (or "parent") nucleic acid. Variant nucleic acids may thus preferably comprise, in their nucleic acid sequence, at least one mutation, substitution, insertion or deletion as compared to their respective reference sequence. Preferably, the term "variant" as used herein includes synthesized or engineered variants of nucleic acid sequences. Therefore, a "variant" as defined herein can be derived from, isolated from, related to, based on or homologous to the reference nucleic acid sequence.

The term "pharmaceutically acceptable" with respect to carriers described herein generally include any and all suitable solid or liquid substances, solvents, dispersion media, coatings, isotonic and absorption delaying agents, and the like that are physiologically compatible with a target protein described herein. Further examples of pharmaceutically acceptable carriers include sterile water, saline, phosphate buffered saline, dextrose, glycerol, ethanol, and the like, as well as combinations of any thereof.

According to one aspect, the pharmaceutical preparation described herein comprises one or more carriers appropriate for a desired route of administration. Such carriers preferably do not reduce the solubilization of the protein through binding of the oligonucleotide to its cognate target. Target proteins and respective complexes may be, e.g. admixed with any of lactose, sucrose, starch, cellulose esters of alkanoic acids, stearic acid, talc, magnesium stearate, magnesium oxide, sodium and calcium salts of phosphoric and sulphuric acids, acacia, gelatin, sodium alginate, polyvinylpyrrolidine, polyvinyl alcohol, and optionally further tableted or encapsulated for conventional administration. Alternatively, a target protein or the protein composition may be dissolved in saline, water, polyethylene glycol, propylene glycol, carboxymethyl cellulose colloidal solutions, ethanol, corn oil, peanut oil, cotton-seed oil, sesame oil, tragacanth gum, and/or various buffers. Other carriers, adjuvants, and modes of administration are well known in the pharmaceutical arts. A carrier may include a controlled release material or time delay material, such as glyceryl monostearate or glyceryl distearate alone or with a wax, or other materials well known in the art.

Additional pharmaceutically acceptable carriers are known in the art and described in, e.g. REMINGTON'S PHARMACEUTICAL SCIENCES. Liquid formulations can be solutions, emulsions or suspensions and can include excipients such as suspending agents, solubilizers, surfactants, preservatives, and chelating agents.

Pharmaceutical compositions are contemplated wherein a protein composition described herein and one or more (further) therapeutically active agents are formulated. Stable formulations are prepared for storage by mixing said protein composition described herein having the desired degree of purity with optional pharmaceutically acceptable carriers, excipients or stabilizers, in the form of lyophilized formulations or aqueous solutions. The formulations to be used for *in vivo* administration are specifically sterile, preferably in the form of a sterile aqueous solution. This is readily accomplished by filtration through sterile filtration membranes or other methods. The target protein or the respective protein composition described herein may also be formulated as immunoliposomes, and/or entrapped in microcapsules.

Administration of the pharmaceutical composition described herein, may be done in a variety of ways, including orally, subcutaneously, intravenously, intranasally, intraotically, transdermally, mucosal, topically, e.g., gels, salves, lotions, creams, etc., intraperitoneally, intramuscularly, intrapulmonary, vaginally, parenterally, rectally, or intraocularly.

Exemplary formulations as used for parenteral administration include those suitable for subcutaneous, intramuscular or intravenous injection as, for example, a sterile solution, emulsion or suspension.

The term "unfolded tertiary structure" with regard to a linear single-stranded oligonucleotide such as described herein, is understood as follows. Single-stranded RNA oligonucleotides may comprise a secondary structure defined by base-paired and unpaired nucleotides from which arise helical duplexes, helical stems, single-stranded regions (hairpins, bulges, internal loops and n-way junctions) and other secondary-structure elements. Base pairs are either canonical (Watson-Crick or wobble base pairs) or non-canonical (formed by edge-to-edge hydrogen bonding interactions between the bases). RNA tertiary structure is then defined as a folded three-dimensional arrangement of such RNA secondary-structure elements kept together via connections called "tertiary interactions", which include long-range Watson-Crick base-pairing, coaxial stacking and A-minor interactions (Butcher and Pyle, Accounts of Chemical Research, 2011, 44, 1302-1311). The tertiary arrangement allows long-range intramolecular RNA interactions, including junctions, kissing loops, pseudoknots, quadruplexes and A-minor motifs (Butcher and Pyle, Accounts of Chemical Research, 2011, 44, 1302-1311), and underlies the recognition of proteins and other molecules with high binding affinities.

An oligo with an unfolded tertiary structure is particularly understood to have an unfolded tertiary structure throughout the whole molecule and is characterized by a dynamic structure that is absent of any stabilizing three-dimensional (tertiary) interactions, but can include formed secondary-structure elements. The absence of a three-dimensional (tertiary) fold can be determined by using biophysical and biochemical techniques including but not limited to nuclear magnetic resonance (NMR), small-angle X-ray scattering, fluorescence resonance energy transfer, chemical probing and computational prediction and modelling.

The three-dimensional (tertiary) structures of RNA are primarily determined through experimental measurements, involving high-resolution techniques such as nuclear magnetic resonance (NMR) and X-ray crystallography in combination with computational modeling, as described by Eric Ennifar (editor) in "Nucleic Acid Crystallography: Methods and Protocols", 2016 (ISBN-10: 1493946412) and Barnawal et al. (Archives of Biochemistry and Biophysics, 2017, 628, 42-56), and low-resolution techniques such as hydroxyl radical footprinting, multiplexed hydroxyl radical cleavage and small-angle X-ray scattering as described by Parisien and Major (Journal of Structural Biology, 2012, 179, 252-260).

Though there are typically oligonucleotide duplex-simplex equilibria depending on the presence of buffer or media, the unfolded tertiary structure of an RNA oligo is herein understood to be determined under physiological conditions e.g., in phosphate buffer at pH 7.4 and 37°C.

The invention specifically provides for exemplary target proteins, oligonucleotide design or the respective protein composition described herein as detailed in the examples provided herein. The foregoing description will be more fully understood with reference to the following examples. Such examples are, however, merely representative of methods of practicing one or more embodiments of the present invention and should not be read as limiting the scope of invention.

### EXAMPLES

### Example 1: Human insulin aggregation assay

### Human insulin sequence

| | |
|---|---|
| α-chain: | GIVEQCCTSICSLYQLENYCN (SEQ ID NO:6) |
| β-chain: | FVNQHLCGSHLVEALYLVCGERGFFYTPKT (SEQ ID NO:7), the target region is underlined (VEALYLVCGERG, SEQ ID NO:8); |

M_{w} = 5722 g/mol, charge = -2

### Aggregation assay

The insulin aggregation assay was performed in 150 mM Tris-HCl pH 7.5. Aggregation was monitored at 360 nm (excitation: 1 nm bandwidth, emission: 10 nm bandwidth) in macro-cuvettes (1500 µl) with a pathlength of 10 mm. 86 µM human insulin (SIGMA) with or without 20 µM RNA were pre-incubated at 30°C and the aggregation reaction was started by the addition of dithiothreitol (DTT) to a final concentration of 15 mM.

### References

Haslbeck et al. (1999) EMBO J. 18, 6744-6751
Kumar & Balbach (2017) Sci. Rep. 7, 42141

**Table 1. Solubility effect of 36 nt homo-oligonucleotides and insulin mRNA fragments**

| **name** | **sequence** | **%G** | **%A** | **%C** | **%U** | **relative solubility** |
|---|---|---|---|---|---|---|
| RNA1* | GUGGAGGCGCUGUACCUGGUGUGCGGAGAGCGCGGC | 50 | 11 | 22 | 17 | **1,21** |
| RNA2 | GUAGAAGCACUAUACCUAGUAUGCGGAGAAAGAGGA | 31 | 39 | 14 | 17 | 0,94 |
| A24 | AAAAAAAAAAAAAAAAAAAAAAAA | 0 | 100 | 0 | 0 | 1,10 |
| C24 | CCCCCCCCCCCCCCCCCCCCCCCC | 0 | 0 | 100 | 0 | 0,84 |
| U24 | UUUUUUUUUUUUUUUUUUUUUUUU | 0 | 0 | 0 | 100 | 1,09 |

| | | | | | | |
|---|---|---|---|---|---|---|
| const. SD 22% | | | | | | |

*RNA1 - a fragment of native own mRNA coding for the potential aggregation region in the human insulin β-chain sequence (VEALYLVCGERG, SEQ ID NO:8);
RNA2 - a re-coded adenine-rich mRNA fragment coding for VEALYLVCGERG (SEQ ID NO:8) peptide
Sequences included in Table 1:

| | |
|---|---|
| RNA1: | SEQ ID NO:4 |
| RNA2: | SEQ ID NO:5 |
| A24: | SEQ ID NO:9 |
| C24: | SEQ ID NO:10 |
| U24: | SEQ ID NO:11 |

### Description of results

The effect of RNA oligonucleotide on solubility of native human insulin was tested in the aggregation assay as described above. While oligonucleotides of simple composition (A24, C24, U24) did not display any visible increase in the solubility of insulin (**Table 1**), the specifically designed RNAs (RNA1, RNA2) affect the solubility of insulin in a predefined way.

RNA1 is compositionally identical to the native coding fragment of the target protein region with 100% sequence identity.

RNA2 is characterized by higher A and lower G content as compared to the reference (RNA1) by 28% and 19%, respectively.

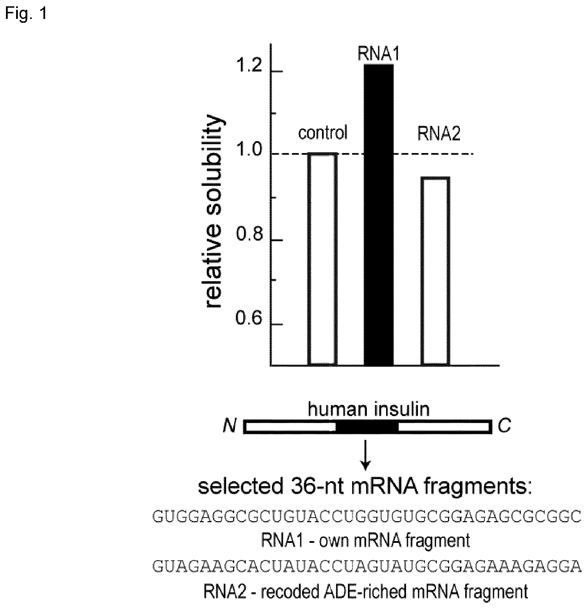

RNA1 provides an increase in the relative solubility by 21 %; RNA2 displays no positive effect (**Table 1,** **Figure 1**). RNA1 was designed based on the native coding mRNA sequence of human insulin using unique computational approach (Nucleic Acids Research 2013, 41(18):8434-8443; FEBS Letters 2018, 592:2901-2916) for identifying protein and mRNA sequence regions with best matching between individual nucleotide composition and corresponding specific amino-acid affinities (particularly, for guanine, G). A twelve amino-acid peptide (VEALYLVCGERG, SEQ ID NO:8) was selected from the β-chain of human insulin and the corresponding coding mRNA fragment (RNA1) was used to test the effects on insulin solubility. RNA2 was designed starting from the RNA1 sequence by introducing a maximum possible number of adenine (A) nucleotides, while keeping the coding relation, in order to perturb affinity to the target region. RNA1, which improves the solubility of human insulin, is compositionally identical to the native coding fragment of the target protein region with 100% sequence identity. In contrast, the ineffective RNA2 is higher in A and lower in G content as compared to the reference (RNA1) by 28% and 19%, respectively i.e. is out of the range as described herein (**Table 1**). The solubility increase for native human insulin in the presence of RNA1 was observed at a molar ratio between protein and RNA of 4.2 : 1, respectively (86 µM : 20 µM) and demonstrates the potential of RNA1 as a solubilizing excipient.

### Example 2: Solubility assay for an aggregation prone region of therapeutic mAb Herceptin

A potential aggregation prone region was selected using state-of-the-art prediction computer program CamSol (Sormanni et al., Sci. Rep. 7, 8200, 2017) for the analysis of *Herceptin* sequence and spatial structure (Cho et al. Nature, 421, 756-60, 2003). The corresponding peptide fragment was identified in *Herceptin* light-chain sequence (46-55) - LLIYSASFLY (SEQ ID NO:1) and was used in further experimental testing by micro BCA protein assay for determination of peptide concentration. An experimental protocol is given below.

### Peptide sequence

Ac-LLIYSASFLY-NH₂ (Trastuzumab/Herceptin), SEQ ID NO:1
M_{w} = 1230.46 g/mol, charge = 0

### Buffer preparation

Phosphate buffer (50 mM in ultrapure water at pH 7.0) was prepared according to the protocol below.

To prepare the phosphate buffer, solutions A and B were made with ultrapure water (Sartorius Stedim Biotech, arium pro VF). Both solutions were filtered through a 0.2 µm filter with a vacuum pump. Afterwards, solutions A and B were stored in the fridge and could be used to produce phosphate buffer at a given pH value. If there was any precipitation, solutions A and B were discarded and prepared anew.
Solution A 0.2 M Na₂HPO₄ (M=141.96 g/mol) from Fisher Bioreagents Solution B 0.2 M NaH₂PO₄ (M=119.98 g/mol) from Fisher Bioreagents
Solution A: for 100 ml at a concentration of 0.2M: 2.8392 g of Na₂HPO₄
Solution B: for 100 ml at a concentration of 0.2M: 2.3996 g of NaH₂PO₄
For 50 ml of 50 mM Phosphate Buffer at pH 7 (25°C), mix: 7.625 ml Solution A + 4.875ml Solution B + 37.5 ml ultrapure water

### Sample preparation

The peptide (LLIYSASFLY, SEQ ID NO:1, purchased from Genscript) was warmed at room temperature. The required amount of peptide was weighed in the Eppi (also pre-weighted peptide-vials, each 500 µg, could be used), the required amount of 50mM PB was added and the sample was vortexed extensively.

RNA oligos were diluted in ultrapure water according to manufacturer's directions to reach a concentration of 100 µM (RNA oligos were purchased from Metabion international AG, all sequences a listed in Table 2). After reconstitution, the RNA concentration was measured with Nanodrop. The aliquots were then made and were frozen at -80°C. When performing the experiment, the RNA was thawed and a sample at a desired concentration was prepared. RNA control samples were made with PB and RNA oligos only (without peptide), for a total volume of 250 µl for 2 replicates.

RNA oligos and peptides were mixed together extensively (total volume: 350 µl for 3 replicates) and vortexed.

### Sonication & Centrifugation

Afterwards, the samples were incubated for 1.5 h in an ultrasonication bath (Sonorex Super 10P, Bandelin). Ice was changed regularly to keep the temperature under 35°C (ideally around 25-28°C). After sonication, the samples were centrifuged for 30 min at 16000 g.

### Micro-BCA Assay

Then, with the supernatants, the Micro-BCA Assay (Thermo Scientific, REF 23235, LOT: TH270461) 2qs performed. The BSA standards ranging from 0 µg/ml to 40 µg/ml were prepared, with two replicates of the standard (each 100 µl) prepared per measurement. Each sample was divided into 3 replicates (350 µl in the beginning, 3*100µl for the assay). For the RNA samples, two replicates were made. For the preparation of the standard and the working reagent, all instructions from Thermo Scientific were followed. The samples and standards were added to the plate and then 100 µl of working reagent was added. The plate was shaken for 30 s and then incubated at 37°C for 2 h. The absorbance was then measured with the Microplate reader TECAN F500 at excitation λ = 595 nm.

The remaining peptide-pellet in the tube was washed with 90 µl PB and centrifugated for 30 min. The supernatant was discarded and then another RNA concentration could be used.

### Overview of the Procedure:

1.) Vortex the sample properly
2.) Ultra-sonicate the samples in ultrasonication bath (SONOREX P10) with 100% power setting for 1.5h
   (Use the floating rack for microtubes and cool the sonicate bath with 1 l beaker filled with ice).
3.) Separate aggregates from dissolved peptide with centrifugation at maximum g (app. 16 000g) for 30 min.
4.) Transfer the liquid phase/supernatant of dissolved peptide in 50 mM PP into micro plate.
5.) Prepare the microplates with loaded 100 µl BSA standards (BSA concentration: 0 - 40 µg/ml) and 100 µl samples with unknown concentration. Incubate microplate at 37°C for 2 h.

### Data Analysis

For data analysis, the absorbance values were imported into Microsoft Excel. The average of each standard was calculated and the 0 µg/ml absorbance value was subtracted as a blank from all other standards. Then the standard curve was plotted and the equation read out.

For the RNA samples, the average values (for the two replicates) were calculated.

These average values were then subtracted from the absorbance values of the samples (similar to a blank, as RNA also shows absorbance in the Micro-BCA Assay). Then the average of these values was calculated and the respective concentration of the sample was calculated with the equation obtained from the standard curve.

If the sample did not include RNA, the 0 µg/ml standard-value was subtracted from the sample and then average concentrations were calculated.

The same protocol was applied to test the solubility effect of single nucleotides and their mixtures (GTP, ATP, CTP, UTP).

### References

Cho et al. (2003) Nature, 421, 756-60
Sormanni et al. (2017) Sci. Rep. 7, 8200

**Table 2. Tested RNA oligo sequences**

| **name** | **sequence** |
|---|---|
| A15 | AAAAAAAAAAAAAAA |
| GGU5 | GGUGGUGGUGGUGGU |
| U15 | UUUUUUUUUUUUUUU |
| C15 | CCCCCCCCCCCCCCC |
| A24 | AAAAAAAAAAAAAAAAAAAAAAAA |
| GGU8 | GGUGGUGGUGGUGGUGGUGGUGGU |
| U24 | UUUUUUUUUUUUUUUUUUUUUUUU |
| C24 | CCCCCCCCCCCCCCCCCCCCCCCC |
| A30 | AAAAAAAAAAAAAAAAAAAAAAAAAAAAAA |
| GGU10 | GGUGGUGGUGGUGGUGGUGGUGGUGGUGGU |
| U30 | UUUUUUUUUUUUUUUUUUUUUUUUUUUUUU |
| C30 | CCCCCCCCCCCCCCCCCCCCCCCCCCCCCC |
| A36 | AAAAAAAAAAAAAAAAAAAAAAAAAAAAAAAAAAAA |
| GGU12 | GGUGGUGGUGGUGGUGGUGGUGGUGGUGGUGGUGGU |
| U36 | UUUUUUUUUUUUUUUUUUUUUUUUUUUUUUUUUUUU |
| C36 | CCCCCCCCCCCCCCCCCCCCCCCCCCCCCCCCCCCC |
| 161811U | UUGUUGAUAUAUAGUGCGAGUUUUUUGUAU |
| 446588C | CUCCUCAUCUACUCCGCCUCCUUCCUCUAC |
| 746496av | CUGCUGAUAUACAGCGCGAGCUUCCUGUAC |
| 745271av | CUGCUGAUAUACUCGGCGUCAUUCCUGUAU |

Sequences included in Table 2:

| | |
|---|---|
| A15: | SEQ ID NO:13 |
| GGU5: | SEQ ID NO:14 |
| U15: | SEQ ID NO:15 |
| C15: | SEQ ID NO:16 |
| A24: | SEQ ID NO:9 |
| GGU8: | SEQ ID NO:12 |
| U24: | SEQ ID NO:11 |
| C24: | SEQ ID NO:10 |
| A30: | SEQ ID NO:17 |
| GGU10: | SEQ ID NO:18 |
| U30: | SEQ ID NO:19 |
| C30: | SEQ ID NO:20 |
| A36: | SEQ ID NO:21 |
| GGU12: | SEQ ID NO:22 |
| U36: | SEQ ID NO:23 |
| C36: | SEQ ID NO:24 |
| 161811U: | SEQ ID NO:2 |
| 446558C: | SEQ ID NO:25 |
| 746496av: | SEQ ID NO:26 |
| 745271av: | SEQ ID NO:27 |

### Description of results

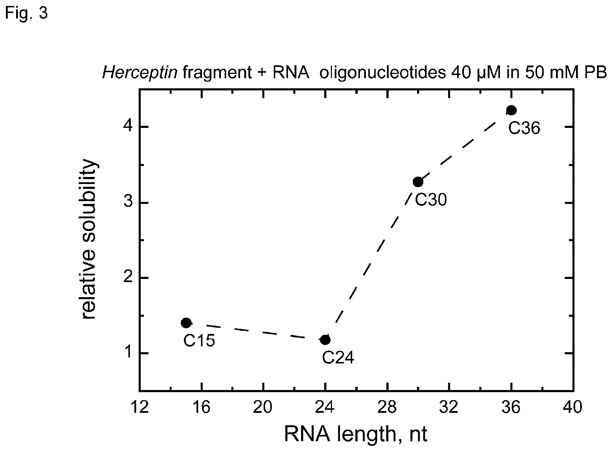

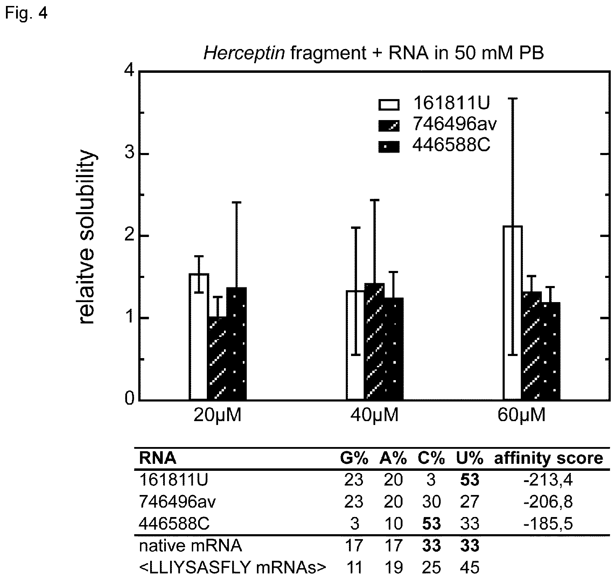

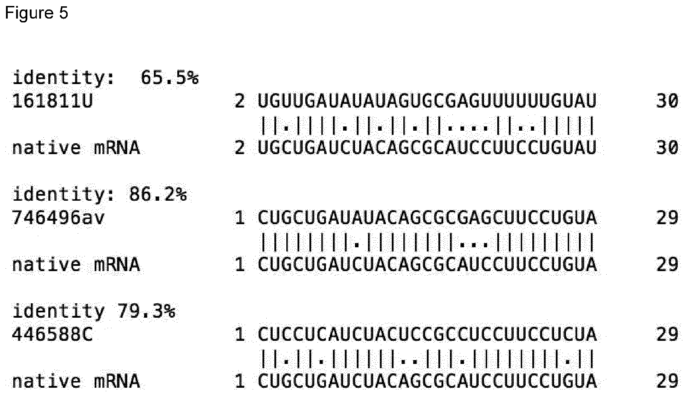

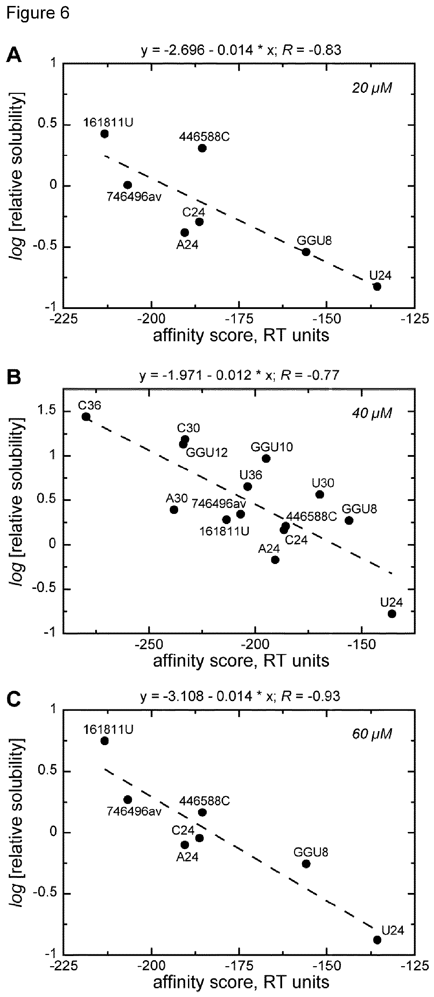

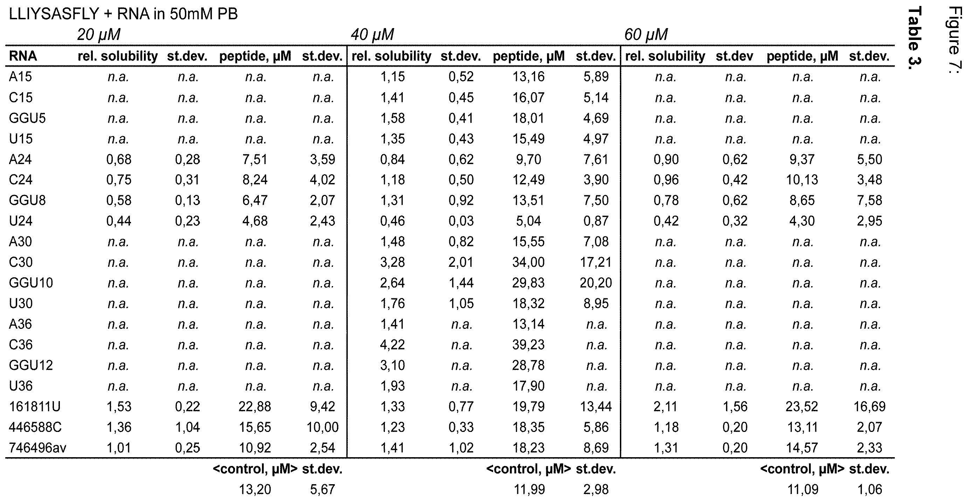

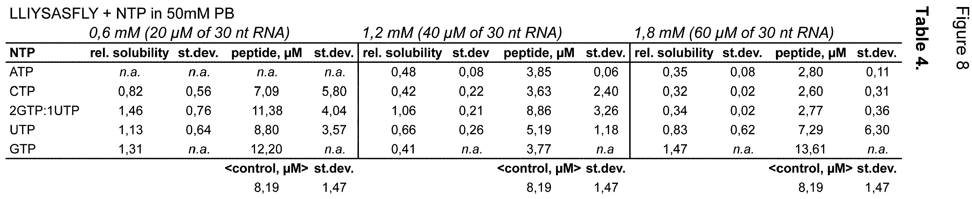

The solubilization effect of RNA oligonucleotides of different length and content **(Table 2)** was tested for an aggregation prone region, with a sequence LLIYSASFLY (SEQ ID NO:1), from a commercial antibody *Herceptin.* First, RNA oligonucleotides showed on average a concentration dependent effect on the relative solubility of the target peptide **(****Figure 2A****)** going from up to 50% at 20 µM RNA concentration, peaking up to 150% at 40 µM and decreasing to a maximum value of 50% at 60 µM. In contrast, single nucleotides (NTP) demonstrated a small positive effect only at 0.6 mM (equivalent of 20 µM RNA, **Figure 2B****,** **Figure 8****(Table 4)).** The results also show that single-stranded linear 30 nt RNA oligonucleotides at the optimal concentration (40 µM in the tested range) are a much more efficient solubilizing agent as compared to equivalent solutions of just single nucleotides (**Figure 2C**), while the increase in the relative solubility of the target peptide depends on the composition of the oligonucleotide used. Among 30 nt long RNAs tested at 40 µM, the strongest effect was observed for C30 homo-oligonucleotide (**Figure 2C****,** **Figure 7** **(Table 3)**) with an average increase between the replicates of 230% (3.3-fold change). The solubilization effect of C homo-oligonucleotide displays dependence on its length (going from 40% (1.4) for 15 nt, 20% (1.2) for 24 nt, 230% (3.3) for 30 nt and 320% (4.2) for 36 nt (**Figure 3****,** **Figure 7** **(Table 3)).** C36 RNA displays the strongest solubilization effect for LLIYSASFLY among all tested RNAs. It increases the ratio between peptide and RNA concentrations ratio from 1 : 3.3 (12 to 40 µM) before mixing to 1 : 1 (40 to 40 µM) upon interaction (**Figure 7** **(Table 3)).** C36 RNA was designed to be composed 100% from the nucleotide enriched in the fragment of *Herceptin* light-chain mRNA fragment coding for LLIYSASFLY (SEQ ID NO:1) peptide **(****Figure 4****).** To test the solubilization effect of specific RNAs, three RNA sequences **(Table 2)** were selected from the pool of all available mRNA fragments coding for LLIYSASFLY (SEQ ID NO:1) peptide (the reference set of sequences) ranked by the affinity score (746496 sequences analyzed in total). The affinity score represents combined affinity values of the target peptide and RNA fragment calculated by unique computational algorithm (FEBS Letters 2018, 592:2901-2916) using specific amino acid / nucleobase affinity scales in water and methanol environments. RNAs with different affinity score values were selected for experimental testing **(****Figure 4****).** The selected mRNAs display high sequence identity (from 66 to 86%) to the native *Herceptin* light-chain mRNA fragment. Their effects on solubility of the target peptide display concentration dependence (**Figure 4****,** **Figure 7** **(Table 3)**) going from an increase by 20 % (1.2-fold change) to 110 % (2.1-fold change). At 60 µM RNA concentration, the observed solubility increase for RNAs labeled as 161811U, 746496av, and 446588C by 110%, 30%, and 20%, respectively, is in accordance with the predicted affinity score values (**Figure 5****,** **Figure 6C**). RNA 161811 U is composed of more than 50% of the most frequent nucleotide in the reference (U, **Figure 4**). RNA 746496av nucleotide content is very similar to that of the native mRNA (deviating anywhere from 3% to 6% in absolute percentage points from the relative fractions of different nucleotides). RNA 446588C is composed of more than 50% of the nucleotide enriched in the native mRNA sequence (C, **Figure 4**). Importantly, the used computational algorithm is able to rank properly the solubilization effects of different RNAs, covering both sequence specificity and length dependence (**Figure 6**). The ranking is shown to be independent of the actual RNA concentration used, which has effect predominantly on the offset of the linear regression.

The native mRNA sequence encoding SEQ ID NO:1 has the following relative content: G - 17%, A - 17%, C - 33%, U - 33% (Fig. 4). The reference - a set of all mRNA sequences coding for LLIYSASFLY (SEQ ID NO:1) - has the following average relative content: G - 11%, A - 19%, C - 25%, U - 45% (Fig. 4).

### Example 3: SLS studies on aggregation of therapeutic mAb Herceptin

Aggregation studies were performed using static light scattering (SLS) techniques. SLS allows monitoring aggregation of biomolecules due to measuring of intensities of the scattered light, which are proportional to the size (mass) of particles in a sample. To illustrate a prevention of aggregation by solubilizing RNAs in a case of a therapeutic mAb, anti-HER2 mAb Trastuzumab/Herceptin was used alone or in a composition with several different RNA oligos designed using the original framework for a predefined aggregation patch in the mAb sequence (see **Example 2**).

### RNA sequences

C30 (SEQ ID NO:20)
161811U (SEQ ID NO:2)
745271av (SEQ ID NO:27),
G - 20%, A - 17%, C - 27%, U - 36%

### Protein sequence

Trastuzumab/Herceptin monomer (664 aa):
>Anti-HER2 Light chain (1 and 2) (SEQ ID NO:28)

DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRTVAAPSV
FIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC

The predefined aggregation patch (aggregation prone region, LLIYSASFLY (SEQ ID NO:1)), is shown in SEQ ID NO:28 of Figure 10 with bold characters.
>Anti-HER2 Heavy chain (1 and 2) (SEQ ID NO:29) 
EVQLVESGGGLVQPGGSLRLSCAASGFNIKDTYIHWVRQAPGKGLEWVARIYPTNGY
TRYADSVKGRFTISADTSKNTAYLQMNSLRAEDTAVYYCSRWGGDGFYAMDYWGQ
GTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTP
PVLDSDGSFFLYSKLTVDKSRWQQGNVFSCSVMHEALHNHYTQKSLSLSPGK
 Mw = 145.5 kg/mol, charge = 12 (for the functional dimer)

### Buffer preparation

Phosphate buffer (PB, 50 mM in ultrapure water at pH 7.0) was prepared according to the protocol given above (**see Example 2**).

### Sample preparation

Herceptin samples with concentration of 1.5 mg/ml in 50 mM PB were prepared using the frozen stock solution of 5 mg/ml in PBS. RNA oligos were diluted in ultrapure water according to manufacturer's directions to reach a concentration of 100 µM (RNA oligos were purchased from Metabion international AG). After reconstitution, the RNA concentration was measured with Nanodrop. The aliquots were then made and were frozen at -80°C. When performing the experiment, the RNA was thawed and samples at 80 µM were prepared.

Protein and RNA samples were spin filtered using a 0.1µm filter at 10,000 g for 4 minutes (Merck Millipore, Ultrafree-MC-W Durapore PVDF) and mixed in a 1:1 ratio. Final concentration for the protein and RNA oligos in the samples for SLS studies was 0.75 mg/ml (5 µM) and 40 µM, respectively that defines protein:RNA molar ratio as 1:8.

### SLS measurements and data analysis

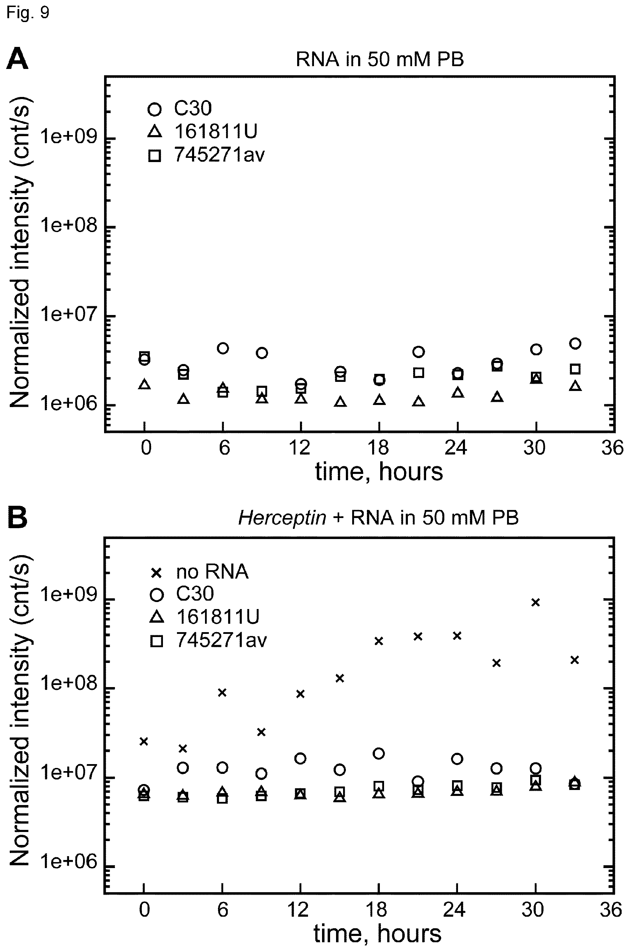

All sample wells were sealed with silica oil to prevent evaporation and data was collected sequentially for approximately 37 hours. Samples were measured in triplicate. All measurements were performed with a WYATT DynaPro PlateReader-II at 37°C in a 1536 well plate (1536W SensoPlate, Greiner Bio-One). For visualization given in Figure 9 normalized intensity values were averaged over replicates within 3 h time window.

### Description of results

As shown below, the presence of tested RNA oligos (C30, 161811U, 745271av) prevent unwanted aggregation of the therapeutic mAb Herceptin for at least 36 h of the experiments. When studying Herceptin alone in the same formulation and concentration (without adding any of the RNA oligos) over 36 h, an increase of scattering intensities was observed in protein samples indicating formation of large (by mass) aggregates (Figure 9B, crosses). At the same time, the tested RNA oligos alone did not display detectable aggregation on the 36 h timescale (Figure 9A).

It can be concluded that RNA oligos that show a solubilizing effect, e.g. Herceptin in a composition with C30, or 16181U (which have a solubilizing effect for the Herceptin aggregation patch, **Example 2**), indeed prevent the formation of large aggregates (increased intensities) as determined by SLS, at least on the 36 h timescale. These two RNA oligos exhibit the same dominant nucleotides (C and U, respectively) as in the native mRNA encoding the Herceptin aggregation patch **(****Figure 4****).** Additionally, no aggregation was observed for Herceptin in a composition with 745271av RNA oligo, which was selected from the library as being close in nucleotide content to the reference. The 745271av relative nucleotide content is very similar to that of the native mRNA encoding the Herceptin aggregation patch (**see Example 2**) and deviates anywhere from 0% to 6% in absolute percentage points from the relative fractions of different nucleotides **(****Figure 4****).** The 745271av and 16181U oligos in a composition with Herceptin display even less fluctuations in intensity indicating a higher level of monodispersity as compared to C30 (**Figure 9B**).

## Claims

1. A composition comprising a protein and at least one single-stranded linear RNA oligonucleotide with an unfolded tertiary structure, which is **characterized by**:
a) a relative content of each of the nucleotides A, C, U, and G, which is the number of each of the nucleotides per total nucleotide number in the oligonucleotide, which relative content is the same ±10% (number of nucleotides per total nucleotide number) as compared to the relative content of the respective nucleotide in a reference; and/or
b) a relative content of at least 50% (number of nucleotides per total nucleotide number) of one type of nucleotide selected from the group consisting of nucleotides A, C, U, and G, which exhibits the highest relative content (number of each of the nucleotides per total nucleotide number) in a reference;
wherein the reference consists of a native mRNA or a set of different RNA sequences that covers all RNA sequences allowed by the universal genetic code, each encoding an amino acid sequence of at least three amino acids within a pre-determined aggregation-prone target region of said protein,
wherein the oligonucleotide has a length which is the same length as the reference, or longer,
wherein solubility of said protein in an aqueous preparation is at least 20% increased as compared to a formulation of said protein that does not comprise said oligonucleotide.

2. The composition of claim 1, wherein the oligonucleotide has a length of 9-100 nt.

3. The composition of claim 1 or 2, wherein the oligonucleotide comprises at least 50% sequence identity to an mRNA encoding said protein.

4. The composition of any one of claims 1 to 3, comprising at least two different single-stranded linear RNA oligonucleotides.

5. The composition of any one of claims 1 to 4, wherein the protein and oligonucleotide(s) are present at a molar ratio ranging between 1:10 and 5:1

6. The composition of any one of claims 1 to 5, which is provided as an aqueous solution, frozen, or in the lyophilized form.

7. An aqueous preparation comprising the composition of any one of claims 1 to 6.

8. The preparation according to claim 7, wherein at least 90% of the protein is monodisperse.

9. The preparation of claim 7 or 8, which does not comprise detectable aggregates of said protein in a static light scattering test as determined at a temperature of 37°C over at least 36 hours.

10. A method of preparing the composition of any one of claims 1 to 6, comprising mixing said protein with said oligonucleotide(s).

11. The method of claim 10, wherein:
a) an aggregation-prone target region of said protein is identified;
b) the average relative content of each of the nucleotides A, C, U, and G, which is the number of each of the nucleotides per total nucleotide number, in a reference consisting of a set of different RNA sequences, each encoding said target region is determined; and
c) at least one of said oligonucleotides is designed and produced, which is **characterized by**
i. a relative content of each of the nucleotides A, C, U, and G, which is the number of each of the nucleotides per total nucleotide number in the oligonucleotide, which relative content is the same as said average relative content of the respective nucleotide ±10% (number of nucleotides per total nucleotide number); and/or
ii. a relative content of at least 50% (number of nucleotides per total nucleotide number) of one type of nucleotide selected from the group consisting of nucleotides A, C, U, and G, which is selected by comparing said average relative content of each of nucleotides A, C, U, and G, and selecting the one type of nucleotide with the highest average relative content.

12. The method of claim 11, wherein said at least one of said oligonucleotides is produced by synthesizing a fragment of an mRNA encoding said target region and optionally modifying the sequence of said mRNA fragment.

13. Use of at least one single-stranded linear RNA oligonucleotide as an excipient in an aqueous protein solution, wherein the oligonucleotide is characterized as in claim 1 and used in an amount sufficient to prevent aggregation of the protein to less than 10% aggregates when stored in solution.

## Patentansprüche

1. Zusammensetzung umfassend ein Protein und zumindest ein einzelsträngiges lineares RNA-Oligonukleotid mit einer ungefalteten Tertiärstruktur, das **gekennzeichnet ist durch**:
a) einen relativen Gehalt eines jeden der Nukleotide A, C, U und G, was die Anzahl eines jeden der Nukleotide je Gesamt-Nukleotidanzahl in dem Oligonukleotid darstellt, wobei der relative Gehalt derselbe ±10 % (Anzahl der Nukleotide je Gesamt-Nukleotidanzahl) im Vergleich zu dem relativen Gehalt des jeweiligen Nukleotids in einer Referenz ist; und/oder
b) einen relativen Gehalt von mindestens 50 % (Anzahl der Nukleotide je Gesamt-Nukleotidanzahl) eines Nukleotid-Typs ausgewählt aus der Gruppe bestehend aus den Nukleotiden A, C, U und G, der den höchsten relativen Gehalt (Anzahl eines jeden der Nukleotide je Gesamt-Nukleotidanzahl) in einer Referenz zeigt;
wobei die Referenz aus einer nativen mRNA oder einem Satz unterschiedlicher RNA-Sequenzen besteht, der alle RNA-Sequenzen abdeckt, die **durch** den universellen genetischen Code zugelassen werden, wobei jede eine Aminosäuresequenz von mindestens drei Aminosäuren innerhalb einer vorbestimmten, zur Aggregation neigenden Zielregion des Proteins kodiert, wobei das Oligonukleotid eine Länge aufweist, welche die gleiche Länge wie die Referenz oder länger ist,
wobei die Löslichkeit des Proteins in einer wässrigen Zubereitung im Vergleich zu einer Formulierung des Proteins, die das Oligonukleotid nicht enthält, um mindestens 20 % erhöht ist.

2. Zusammensetzung nach Anspruch 1, **dadurch gekennzeichnet, dass** das Oligonukleotid eine Länge von 9 - 100 nt aufweist.

3. Zusammensetzung nach Anspruch 1 oder 2, wobei das Oligonukleotid zumindest 50 % Sequenzidentität mit einer mRNA umfasst, welche für das Protein kodiert.

4. Zusammensetzung nach einem der Ansprüche 1 bis 3, umfassend zumindest zwei unterschiedliche einzelsträngige lineare RNA-Oligonukleotide.

5. Zusammensetzung nach einem der Ansprüche 1 bis 4, wobei das Protein und das/die Oligonukleotid(e) in einem molaren Verhältnis zwischen 1:10 und 5:1 vorliegen.

6. Zusammensetzung nach einem der Ansprüche 1 bis 5, die als wässrige Lösung, gefroren oder in lyophilisierter Form bereitgestellt wird.

7. Wässrige Zubereitung, welche die Zusammensetzung nach einem der Ansprüche 1 bis 6 umfasst.

8. Zubereitung nach Anspruch 7, wobei mindestens 90 % des Proteins monodispers sind.

9. Zubereitung nach Anspruch 7 oder 8, das keine nachweisbaren Aggregate des Proteins in einem statischen Lichtstreuungstest umfasst, bestimmt bei einer Temperatur von 37 °C über mindestens 36 Stunden.

10. Verfahren zur Herstellung der Zusammensetzung nach einem der Ansprüche 1 bis 6, umfassend das Mischen des Proteins mit dem/den Oligonukleotid(en).

11. Verfahren nach Anspruch 10, wobei:
a) eine zur Aggregation neigende Zielregion des Proteins identifiziert wird;
b) der relative Gehalt eines jeden der Nukleotide A, C, U und G, was die Anzahl eines jeden der Nukleotide je Gesamt-Nukleotidanzahl darstellt, in einer Referenz bestimmt wird, welche aus einem Satz unterschiedlicher RNA-Sequenzen besteht, die jeweils für die Zielregion kodieren, und
c) zumindest eines der Oligonukleotide konstruiert und produziert wird, **gekennzeichnet durch**
i.) einen relativen Gehalt eines jeden der Nukleotide A, C, U und G, was die Anzahl eines jeden der Nukleotide je Gesamt-Nukleotidanzahl in dem Oligonukleotid darstellt, wobei der relative Gehalt derselbe wie der mittlere relative Gehalt des jeweiligen Nukleotids ±10 % (Anzahl der Nukleotide je Gesamt-Nukleotidanzahl) ist, und/oder
ii. einen relativen Gehalt von mindestens 50 % (Anzahl der Nukleotide je Gesamt-Nukleotidanzahl) eines Nukleotid-Typs ausgewählt aus der Gruppe bestehend aus den Nukleotiden A, C, U und G, der ausgewählt wird, indem der mittlere relative Gehalt eines jeden der Nukleotide A, C, U und G verglichen und der Nukleotid-Typ mit dem höchsten mittleren relativen Gehalt ausgewählt wird.

12. Verfahren nach Anspruch 11, wobei das zumindest eine der Oligonukleotide durch Synthetisieren eines Fragments einer mRNA, welche für die Zielregion kodiert, und gegebenenfalls Modifizieren der Sequenz des mRNA-Fragments hergestellt wird.

13. Verwendung zumindest eines einzelsträngigen linearen RNA-Oligonukleotids als Exzipient in einer wässrigen Proteinlösung, wobei das Oligonukleotid wie in Anspruch 1 gekennzeichnet ist und in einer Menge verwendet wird, die ausreichend ist, um die Aggregation des Proteins auf weniger als 10 % Aggregate zu verhindern, wenn es in Lösung gelagert wird.

## Revendications

1. Composition comprenant une protéine et au moins un oligonucléotide d'ARN linéaire simple brin avec une structure tertiaire dépliée, qui est **caractérisée par** :
a) une teneur relative de chacun des nucléotides A, C, U et G, qui est le nombre de chacun des nucléotides par rapport au nombre total de nucléotides dans l'oligonucléotide, laquelle teneur relative est la même à ±10 % (nombre de nucléotides par rapport au nombre total de nucléotides) compararativement à la teneur relative du nucléotide respectif dans une référence ; et/ou
b) une teneur relative d'au moins 50 % (nombre de nucléotides par rapport au nombre total de nucléotides) d'un type de nucléotide choisi dans le groupe constitué par les nucléotides A, C, U et G, qui présente la teneur relative la plus élevée (nombre de chacun des nucléotides par rapport au nombre total de nucléotides) dans une référence ;
dans laquelle la référence se compose d'un ARN natif ou d'un ensemble de séquences d'ARN différentes qui couvre toutes les séquences d'ARN autorisées par le code génétique universel, codant chacun une séquence d'acides aminés d'au moins trois acides aminés avec une région cible prédisposée à l'agrégation prédéfinie de ladite protéine,
dans laquelle l'oligonucléotide présente une longueur qui est la même longueur que la référence, ou plus longue,
dans laquelle la solubilité de ladite protéine dans une préparation aqueuse est accrue d'au moins 20 % comparativement à une formulation de ladite protéine qui ne comprend pas ledit oligonucléotide.

2. Composition selon la revendication 1, dans laquelle l'oligonucléotide présente une longueur de 9 à 100 nt.

3. Composition selon la revendication 1 ou 2, dans laquelle l'oligonucléotide comprend une identité de séquence d'au moins 50 % avec un ARNm codant ladite protéine.

4. Composition selon l'une quelconque des revendications 1 à 3, comprenant au moins deux oligonucléotides d'ARN linéaires simple brin différents.

5. Composition selon l'une quelconque des revendications 1 à 4, dans laquelle la protéine et le(s) oligonucléotide(s) est/sont présent(s) en un rapport molaire compris entre 1:10 et 5:1.

6. Composition selon l'une quelconque des revendications 1 à 5, qui est fournie sous la forme d'une solution aqueuse, congelée ou sous la forme lyophilisée.

7. Préparation aqueuse comprenant la composition selon l'une quelconque des revendications 1 à 6.

8. Préparation selon la revendication 7, dans laquelle au moins 90 % de la protéine est monodispersée.

9. Préparation selon la revendication 7 ou 8, qui ne comprend pas d'agrégats détectables de ladite protéine dans un test de dispersion de la lumière statique tel que déterminé à une température de 37°C pendant au moins 36 heures.

10. Procédé de préparation de la composition selon l'une quelconque des revendications 1 à 6, comprenant le mélange de ladite protéine avec le(s)dit(s) oligonucléotide(s).

11. Procédé selon la revendication 10, dans lequel :
a) une région cible prédisposée à l'agrégation de ladite protéine est identifiée ;
b) la teneur relative moyenne de chacun des nucléotides A, C, U et G qui est le nombre de chacun des nucléotides par rapport au nombre total de nucléotides, dans une référence constituée d'un ensemble de séquences d'ARN différentes, codant chacun ladite région cible est déterminée ; et
c) au moins l'un desdits oligonucléotides est conçu et produit, qui est **caractérisé par**
i) une teneur relative de chacun des nucléotides A, C, U et G, qui est le nombre de chacun des nucléotides par rapport au nombre total de nucléotides dans l'oligonucléotide, laquelle teneur relative est la même que ladite teneur relative moyenne du nucléotide respectif à ±10 % (nombre de nucléotides par rapport au nombre total de nucléotides) ; et/ou
ii) une teneur relative d'au moins 50 % (nombre de nucléotides par rapport au nombre total de nucléotides) d'un type de nucléotide choisi dans le groupe constitué par les nucléotides A, C, U et G, qui est choisie en comparant ladite teneur relative moyenne de chacun des nucléotides A, C, U et G, et en choisissant le type de nucléotide avec la teneur relative moyenne la plus élevée.

12. Procédé selon la revendication 11, dans lequel ledit au moins un desdits oligonucléotides est produit par la synthèse d'un fragment d'un ARNm codant ladite région cible et éventuellement la modification de la séquence dudit fragment d'ARNm.

13. Utilisation d'au moins un oligonucléotide d'ARN linéaire simple brin sous la forme d'un excipient dans une solution aqueuse de protéine, dans laquelle l'oligonucléotide est caractérisé comme dans la revendication 1 et utilisé en une quantité suffisante pour empêcher l'agrégation de la protéine à moins de 10 % d'agrégats lorsqu'elle est stockée en solution.
